(19) Europäisches Patentamt / European Patent Office / Office européen des brevets

(11) **EP 2 376 925 B1**

(12) **FASCICULE DE BREVET EUROPEEN**

(45) Date de publication et mention
de la délivrance du brevet:
**14.01.2015 Bulletin 2015/03**

(21) Numéro de dépôt: **09801230.5**

(22) Date de dépôt: **15.12.2009**

(51) Int Cl.:
***G01N 33/68*** *(2006.01)*

(86) Numéro de dépôt international:
**PCT/FR2009/001424**

(87) Numéro de publication internationale:
**WO 2010/076413 (08.07.2010 Gazette 2010/27)**

(54) **PROCEDE D'EVALUATION DE L'IMMUNOGENICITE DES PROTEINES**

VERFAHREN ZUR BEWERTUNG DER IMMUNOGENITÄT VON PROTEINEN

METHOD FOR EVALUATING THE IMMUNOGENICITY OF PROTEINS

(84) Etats contractants désignés:
**AT BE BG CH CY CZ DE DK EE ES FI FR GB GR
HR HU IE IS IT LI LT LU LV MC MK MT NL NO PL
PT RO SE SI SK SM TR**

(30) Priorité: **18.12.2008 FR 0807123**

(43) Date de publication de la demande:
**19.10.2011 Bulletin 2011/42**

(73) Titulaires:
• **Proteus
30000 Nîmes (FR)**
• **Commissariat à l'Énergie Atomique
et aux Énergies Alternatives
75015 Paris (FR)**

(72) Inventeurs:
• **MAILLERE, Bernard
F-78000 Versailles (FR)**
• **DELLUC-DESROCHES, Stéphanie
F-28700 La Chapelle d'Aunainville (FR)**

(74) Mandataire: **Leblois-Préhaud, Hélène Marthe
Georgette
Cabinet Orès
36, rue de St Pétersbourg
75008 Paris (FR)**

(56) Documents cités:
**WO-A-2006/042333**

• **JABER ET AL: "Assessment of the
immunogenicity of different interferon beta-1a
formulations using ex vivo T-cell assays"
JOURNAL OF PHARMACEUTICAL AND
BIOMEDICAL ANALYSIS, NEW YORK, NY, US,
vol. 43, no. 4, 23 février 2007 (2007-02-23), pages
1256-1261, XP005901641 ISSN: 0731-7085**
• **DE GROOT ET AL: "Immunogenicity of protein
therapeutics" TRENDS IN IMMUNOLOGY,
ELSEVIER, RAHWAY, NJ, US, vol. 28, no. 11, 25
octobre 2007 (2007-10-25), pages 482-490,
XP022342838 ISSN: 1471-4906**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

La présente invention concerne un procédé permettant d'évaluer l'immunogénicité des protéines chez l'homme ou l'animal qui repose sur l'analyse de la réponse lymphocytaire T CD4+ spécifique de la protéine à tester chez des individus de l'espèce (humaine ou animale) dans laquelle l'immunogénicité de ladite protéine est analysée.

Les protéines ont la particularité d'être potentiellement immunogènes c'est-à-dire d'être capables de déclencher une réponse immunitaire dirigée contre elles mêmes. Selon l'activité thérapeutique attendue, la réponse immunitaire dirigée contre une protéine peut être bénéfique ou au contraire indésirable. Elle est bien évidemment souhaitée quand la protéine est un candidat vaccin alors qu'elle est redoutée lorsque la protéine possède une activité thérapeutique indépendante de la réponse immunitaire.

Les protéines représentent depuis 1995 un tiers des produits thérapeutiques mis sur le marché. Elles présentent de nombreux avantages notamment de spécificité, comme par exemple les anticorps monoclonaux qui ciblent des récepteurs ou les vaccins qui induisent des réponses immunitaires spécifiques d'agents pathogènes. Les réponses immunitaires induites par les protéines ont des conséquences très variables d'une protéine à une autre.

Dans certains cas, les anticorps induits peuvent diminuer l'efficacité de la protéine (facteur VIII de coagulation, Interféron béta (IFN-beta)). Dans d'autres cas, ils peuvent poser des problèmes de santé comme par exemple l'érythropoïétine ou la thrombopoïétine pour lesquelles les anticorps induits neutralisent la molécule endogène et provoquent des effets opposés à ceux attendus. Les protéines peuvent être allergisantes et provoquer des allergies dont l'intensité varie de symptômes locaux désagréables à des crises systémiques de type anaphylactique pouvant mettre en danger la vie du patient.

En revanche, des candidats vaccins peuvent induire des réponses immunitaires insuffisantes pour être efficaces ou chez un nombre limité de patients.

Enfin, les outils modernes d'ingénierie des protéines et d'évolution dirigée telle que le L-Shuffling permettent de générer des protéines ayant des séquences originales et des activités biologiques accrues. L'immunogénicité de ces nouvelles protéines modifiées n'est pas connue.

L'immunogénicité d'une solution d'une protéine ne résulte pas seulement de la protéine elle-même mais peut également résulter de facteurs extrinsèques (A.S. de Groot et D. W. Scott, TRENDS in Immunology, 27 octobre 2007, Vol. 28). Par exemple, les molécules contaminantes présentes dans les préparations de protéines, par exemple l'ADN bactérien (CpG) et les endotoxines contaminants les préparations de protéines recombinantes, ainsi que les aggrégats de protéines peuvent favoriser une réponse immunitaire contre une préparation d'une protéine En outre, les modifications des protéines comme la modification de la séquence en acides aminés (mutations), la dénaturation, les modifications post-traductionnelles (glycosylation par exemple) et la complexation ou le couplage à des composés organiques (pegylation, par exemple) peuvent, en fonction de leur nature, soit augmenter, soit diminuer l'immunogénicité des protéines.

Ces problèmes mettent en évidence l'importance de prévoir l'immunogénicité des protéines avant qu'elles ne soient administrées à l'homme ou à l'animal et d'évaluer l'immunogénicité de préférence sur la solution de la protéine qui sera injectée

En outre, des protéines non-thérapeutiques (protéines humaines pour l'homme ou de la même espèce pour l'animal) peuvent devenir immunogènes suite à une modification de leur structure par des composés organiques, notamment des composés thérapeutiques. En effet, la complexation ou le couplage de protéines (sérum albumine humaine, par exemple) à certaines molécules thérapeutiques (haptène) ou à leurs métabolites haptèniques (groupe penicilloyl des antibiotiques de la famille des béta-lactames) peut survenir *in vivo* après administration des molécules organiques et peut déclencher chez l'homme des réactions immunitaires pouvant aller jusqu'à des réponses allergiques (U. Kragh-Hansen, Pharmacol. Rev., 1981, 33, 17-53 ; H. Bundgaard et J. Hansen, J. Pharm. Pharmacol., 1982, 34, 304-309 ; Lafaye et C. Lapresle, J. Clin. Invest., 1988, 82, 7-12 ; Brander et al., The Journal of Immunology, 1995, 155, 2670-2678 ; P; J. P. Thyssen et H.I. Maibach, Contact Dermatitis, 2008, 59, 195-202). Ces réponses sont dépendantes des lymphocytes T et spécifiques des composés. En fait, les cellules T les reconnaissent sous la forme de peptides modifiés. Ces peptides sont soit directement modifiés en surface de la cellule présentatrice d'antigène sur la molécule HLA, soit issus de la dégradation de protéines modifiées.

En conséquence, il est également important de prévoir l'immunogénicité de protéines modifiées par des composés thérapeutiques avant que ces composés ne soient administrés à l'homme ou à l'animal.

Les réponses immunitaires contre les protéines font intervenir trois types cellulaires différents qui en coopérant aboutissent à la production d'anticorps spécifiques des protéines.

Les lymphocytes B sont les cellules qui produisent les anticorps. Chaque lymphocyte B produit une seule molécule d'anticorps. La séquence de cet anticorps résulte de la recombinaison des gènes d'immunoglobulines qui se produit dans chaque précurseur de lymphocyte B. Compte tenu du nombre élevé de gènes d'immunoglobulines et de la combinatoire des réarrangements, un important répertoire d'anticorps est ainsi produit et permet de reconnaître la majorité des molécules présentées au système immunitaire. Les lymphocytes B naïfs (c'est-à-dire qui n'ont jamais vu l'antigène) possèdent une IgM de surface qui leur permet de reconnaître une protéine. Lors du premier contact avec la protéine, sa reconnaissance provoque la réponse primaire caractérisée par la sécrétion d'IgM spécifiques de la protéine. La réponse secondaire apparaît lors des contacts ultérieurs. Elle se caractérise par la sécrétion d'autres classes d'immunoglobulines, notamment des IgG et des IgE spécifiques de la protéine et nécessite l'intervention de lymphocytes T CD4+ également spécifiques de la protéine.

Les cellules dendritiques (DC) sont des cellules présentatrices d'antigènes (CPA) professionnelles qui ont été découvertes par Steinman en 1973 (J. Exp. Med., 1973, 137:1142-1162). Les DC existent sous deux formes: des DC immatures et des DC matures. Les DC immatures ont une forte capacité d'endocytose et jouent le rôle de sentinelles dans les tissus périphériques. Les DC matures sont impliquées dans l'initiation des réponses immunitaires du fait de la présentation par les molécules du complexe majeur d'histocompatibilité (CMH) aux lymphocytes T, des peptides dérivés des antigènes qu'elles ont endocytosés à l'état immature. Les cellules dendritiques expriment le marqueur DC-SIGN ainsi que les molécules du CMH (molécules HLA chez l'homme). Il est possible de différencier les DC matures des DC immatures par des marqueurs de surface. Les DC matures expriment en effet des molécules de co-stimulation telles que le CD83, le CD86 et le CD40 alors que leur expression est moindre sur les DC immatures. La fonction de présentation des peptides est assurée par les molécules CMH de classe I (HLA I chez l'homme) vis-à-vis des lymphocytes T CD8+ et des molécules CMH de classe II (HLA II chez l'homme) vis-à-vis des lymphocytes T CD4+. Il existe quatre types de molécules HLA de classe II : 2 types HLA-DR, 1 type HLA-DQ et 1 type HLA-DP. Ces molécules présentent de fortes homologies de séquence et adoptent une structure commune. Toutefois, elles sont très polymorphes car chaque type de molécule HLA de classe II possède de nombreux allèles dans le monde. En 2008, on comptait 640 allèles DRB, 91 DQB1 et 128 DPB1. Cette diversité intervient directement sur la fonction de présentation de peptides par les molécules HLA de classe II car les variations de séquences sont principalement localisées dans le site de fixation des peptides. Du fait de ces variations de séquences, chaque molécule lie un répertoire de peptides qui lui est propre. C'est pourquoi la localisation des épitopes T CD4+ qui sont les peptides que reconnaissent les lymphocytes T CD4+ dépend des molécules HLA de classe II de chaque individu et varie d'un individu à un autre.

Les lymphocytes T CD4+ sont les principales cellules qui contrôlent l'apparition des anticorps. Ces cellules se caractérisent par l'expression du CD3, propre aux lymphocytes T et le CD4 qui les différencient des lymphocytes T CD8. Il s'agit de cellules spécifiques des protéines. Elles sont en effet spécifiquement activées par les peptides issus des protéines que leur présentent les molécules HLA de classe II. La reconnaissance des complexes molécules HLA de classe II/peptides se fait par le récepteur T. Le récepteur T est un récepteur réarrangé qui possède une structure proche de celle des immunoglobulines. Généré par un réarrangement des gènes de récepteur T, le récepteur T est propre à chaque lymphocyte T. L'activation des lymphocytes T CD4+ se traduit par la sécrétion de cytokines telles que l'IL-2, l'IL-4 ou l'IFN-$\gamma$ et l'apparition de molécules de co-stimulation telles que le CD40L.

La coopération cellulaire entre ces trois types cellulaires aboutit à la production d'anticorps dirigés contre les protéines.

La protéine est captée par les cellules dendritiques immatures et est dégradée en peptides. Parmi les peptides générés, certains possèdent des résidus d'ancrage appropriés pour se fixer sur les molécules CMH de classe II et être présentés aux lymphocytes T CD4+. Sous l'effet de facteurs inflammatoires pouvant résulter de l'administration de la protéine, les cellules dendritiques immatures se différencient en cellules dendritiques matures et deviennent aptes à stimuler des lymphocytes T CD4+ naïfs. Elles migrent dans les ganglions lymphatiques où elles rencontrent les lymphocytes T CD4+. Des lymphocytes T CD4+ possédant un récepteur T spécifique des complexes peptides/molécule CMH de classe II sont activés par la présentation des peptides issus de la protéine et présentés par les molécules CMH de classe II à la surface des cellules dendritiques matures. Ils passent alors d'un état de lymphocyte T CD4+ naïfs à celui de lymphocytes T CD4+ expérimentés ou lymphocytes T CD4+ activés. La protéine parvient également aux lymphocytes B. Les lymphocytes B possédant une immunoglobuline de surface adaptée à la reconnaissance de la protéine internalisent la protéine, la dégradent et génèrent des peptides. Selon un processus similaire à celui décrit dans les cellules dendritiques, des peptides se lient aux molécules CMH de classe II et sont présentés aux lymphocytes T CD4+ spécifiques et expérimentés. La reconnaissance de ces peptides les active et leur font délivrer, par des contacts cellulaires (CD40/CD40L) et par les cytokines qu'ils sécrètent, l'aide nécessaire à la différenciation des lymphocytes B en plasmocytes sécréteurs d'anticorps spécifiques de la protéine. Ces anticorps produits contre la protéine peuvent neutraliser son activité et modifier sa pharmacocinétique.

Les lymphocytes T CD4+ contribuent donc de manière majeure au déclenchement de la réponse humorale contre les protéines. C'est en effet leur recrutement qui permet la sécrétion par les lymphocytes B des anticorps spécifiques des protéines. Plusieurs observations effectuées chez les hémophiles illustrent le rôle majeur des lymphocytes T CD4+ dans l'apparition des anticorps neutralisants. L'hémophilie A est due à une déficience pour le facteur de coagulation VIII et est traitée par des injections régulières de facteur VIII. Plus de 30 % des hémophiles ayant une hémophilie sévère développent une réponse humorale qui inhibe l'action du facteur VIII. Les anticorps inhibiteurs sont des IgG (Jacquemin et al., Blood, 1998, 92:496-506) dont on sait que l'apparition est sous la dépendance des lymphocytes T CD4+. En effet, des mutations sont observées dans les gènes codant pour les parties variables des anticorps anti-facteur VIII (Jacquemin et al., Blood, 1998, 92:496-506). L'apparition de ces mutations est un phénomène dépendant des lymphocytes T CD4+. Chez des sujets hémophiles avec un taux défini d'anticorps anti-Facteur VIII, l'infection par le virus du SIDA provoque la disparition des anticorps spécifiques lorsque le taux de lymphocytes T CD4+ diminue (Bray et al., Am. J. Hematol., 1993, 42:375-379). Du fait du rôle majeur des lymphocytes T CD4+ spécifiques, la capacité d'une protéine à stimuler des lymphocytes T CD4+ naïfs est attendue refléter sa capacité à induire des anticorps. Ainsi, des études effectuées chez l'animal ont montré que des peptides (Herve et al., Mol. Immunol., 1997, 34:157-163) et des protéines (Yeung et al., J. Immunol., 2004, 172:6658-6665) qui après modification de leur séquences ont perdu la faculté d'induire l'activation de lymphocytes T CD4+ naïfs, n'induisent pas d'anticorps (Herve et al., Mol. Immunol., 1997, 34:157-163; Yeung et al., J. Immunol., 2004, 172:6658-6665).

Cette activation est le résultat d'au moins trois différentes phases qui ont chacune une influence sur l'activation des lymphocytes T CD4+ (Adorini et al., J. Exp. Med., 1988, 168:2091-2104):

1. l'apprêtement de la protéine qui comprend sa capture par les cellules dendritiques immatures et sa dégradation en peptides,
2. la fixation des peptides sur les molécules CMH de classe II (HLA II chez l'homme), et
3. la reconnaissance par les lymphocytes T CD4+ naïfs, des peptides présentés par les molécules CMH de classe II (HLA II chez l'homme) à la surface des cellules dendritiques matures.

C'est pourquoi les méthodes d'évaluation de l'immunogénicité des protéines cherchent à reproduire l'activation des lymphocytes T CD4+ naïfs humains.

*Les modèles animaux*

Les modèles animaux sont principalement la souris, le rat, le lapin ou le singe. Pour évaluer l'immunogénicité des protéines, les protéines sont injectées à plusieurs reprises et selon différentes voies d'administration aux animaux et l'apparition des anticorps est recherchée. La prédiction de l'immunogénicité de protéines thérapeutiques est considérée comme faible. En effet, la réponse en anticorps étant sous la dépendance des lymphocytes T CD4+, les différences de spécificité entre les molécules du CMH des animaux et les molécules HLA de classe II de l'homme entraînent des différences importantes d'immunogénicité. La nature et le nombre de peptides présentés par les molécules de CMH de classe II des animaux et reconnus par les lymphocytes T CD4+ sont différents de ceux présentés par les molécules HLA de classe II. De plus, le répertoire en lymphocytes B et en lymphocytes T des animaux est formé par des processus de sélection qui peuvent aboutir à un répertoire différent de celui de l'homme. Des lymphocytes T et B en cours de différenciation sont en effet éliminés de la circulation sanguine par la présentation des molécules du soi. Ces différences mêmes subtiles de composition du répertoire provoquent des différences importantes de réponse en particulier vis-à-vis de protéines humaines.

*Les méthodes in silico*

Les méthodes *in silico* identifient des peptides susceptibles de se fixer aux molécules HLA de classe II et donc tentent de ne reproduire que la deuxième phase du processus (Sturniolo et al., Nat. Biotechnol., 1999, 17:555-561; Koren et al., Clin. Immunol., 2007, 124:26-32). Ces méthodes sont basées sur la présence de motifs spécifiques aux molécules HLA de classe II ou sur des scores de prédiction établis à partir de matrices reflétant l'influence des 20 acides aminés à chaque position sur la liaison ou sur des systèmes experts. D'autres approches sont basées sur une analyse structurale des complexes molécule HLA/peptide (Desmet et al., Proteins, 2005, 58:53-69). Chacune de ces approches génère à la fois des faux positifs et des faux négatifs vis-à-vis de la fixation des peptides sur les molécules HLA de classe II. Une récente étude comparative montre que la valeur prédictive de ces tests pour identifier des peptides ayant une capacité à stimuler les lymphocytes T CD4+ est faible, ce qui signifie que le nombre de faux positifs est élevé et peu satisfaisant (Wang et al., PLoS Comput. Biol., 2008, 4:e1000048).

*Les méthodes biochimiques*

Les méthodes biochimiques *in vitro* identifient des peptides sur la base de leur affinité pour les molécules HLA de classe II. Comme les méthodes *in silico,* ces approches souffrent de ne porter que sur la deuxième phase du processus d'activation des lymphocytes T CD4+. Des peptides se fixant aux molécules HLA de classe II peuvent ne pas induire d'activation des lymphocytes T CD4+ (Castelli et al., Eur. J.Immunol., 2007, 37:1513-1523) si bien que cette méthode souffre également d'un nombre élevé de faux positifs.

*Les méthodes cellulaires*

Il est également possible d'utiliser des tests cellulaires *in vitro* pour évaluer l'immunogénicité des protéines chez l'homme. Ces approches sont basées sur l'activation spécifique de lymphocytes T CD4+ chez les sujets n'ayant jamais été en contact avec l'antigène (Tangri et al., J. Immunol., 2005, 174, 3187-3196; Stickler et al., Environ. Health Perspect., 2003, 111:251-254; Jaber, A. et M. Baker, J Pharm. Biomed Anal., 2007, 43:1256-1261; Brevets EP 1512004, EP 1483581, EP 1073754).

Des cellules présentatrices humaines sont incubées en présence d'antigènes (peptides ou protéines) puis mises en culture avec des lymphocytes CD4+ issus du même donneur. Les lymphocytes T CD4+ spécifiques des antigènes sont stimulés et prolifèrent. Leur nombre augmente si bien qu'ils deviennent détectables par un test cellulaire tel que l'incorporation de thymidine tritiée ou la sécrétion de cytokines. Ces approches tentent donc de reproduire *in vitro* l'activation et l'amplification des lymphocytes T CD4+ spécifiques de la protéine qui pourrait avoir lieu *in vivo* chez l'individu auquel la protéine est destinée à être administrée.

*Les méthodes cellulaires utilisant uniquement des peptides*

Plusieurs méthodes d'évaluation de l'immunogénicité des protéines et d'ingénierie de protéines possédant une immunogénicité réduite sont basées sur l'utilisation de peptides pour stimuler les lymphocytes T CD4+ naïfs et détecter les lymphocytes T CD4+ spécifiques de la protéine ainsi activés (Stickler et al., Environ. Health Perspect., 2003, 111:251-254; Brevets EP 1512004, EP 1483581, EP1073754). Les peptides utilisés couvrent l'ensemble de la séquence de la protéine étudiée. Les avantages d'utiliser des peptides sont leur très bonne réactivité dans les tests d'activation, leur facilité de production et de stockage et la possibilité qu'ils offrent de localiser les séquences immunogéniques. Toutefois, pour évaluer l'immunogénicité d'une protéine ils présentent de nombreux inconvénients. De manière à couvrir le mieux possible la séquence de la protéine, un nombre important de peptides doit être synthétisé et testé ce qui inexorablement augmente le coût du test d'évaluation de l'immunogénicité. De plus, la taille optimale des peptides pour activer des lymphocytes T CD4+ étant très variable d'un épitope T CD4+ à un autre, il est possible que la collection de peptides couvrant la séquence de la protéine ne contienne pas les peptides optimaux et ne permette donc pas de détecter tous les lymphocytes T CD4+ spécifiques de la protéine. Réciproquement, l'utilisation de peptides lors des phases d'activation des lymphocytes T CD4+ risque d'activer des lymphocytes T CD4+ qui ne sont pas spécifiques de la protéine et peut donc générer des réponses faussement positives. En effet, ces approches ne prennent pas en compte l'étape de capture et de dégradation des peptides dans la cellule présentatrice. Les peptides ne nécessitent pas d'apprêtement par la cellule présentatrice et sont chargés sur les molécules HLA présentes à la surface de la cellule présentatrice. Bien qu'étant contenus dans la séquence de la protéine, des épitopes peuvent en fait ne pas être spécifiques de la protéine. De tels épitopes sont appelés épitopes cryptiques (Gammon et al., Immunol. Rev., 1987, 98:53-73). Ils induisent des lymphocytes T CD4+ spécifiques d'eux-mêmes mais ces lymphocytes T CD4+ ne peuvent pas reconnaître la protéine présentée par les cellules présentatrices. Ces épitopes peuvent correspondre à des peptides qui sont dégradés lors de l'apprêtement par la cellule présentatrice (Adorini et al., J. Exp.Med., 1988, 168:2091-2104) ou à des peptides dont la séquence équivalente dans la protéine porte une modification post-traductionnelle qui empêche sa présentation. L'utilisation de peptides lors des phases d'activation des lymphocytes T CD4+ risque donc de surestimer l'immunogénicité des protéines.

De plus, une protéine est issue d'un processus de fabrication défini. Elle peut subir des modifications post-traductionnelles telles que la glycosylation. La nature et l'étendue de la glycosylation dépendent de la cellule hôte servant à la production de la protéine. Au cours de sa fabrication et de son stockage, une protéine peut subir des altérations telles que l'oxydation des méthionines et des tryptophanes. Enfin elle peut contenir des molécules contaminantes qui participent à l'immunogénicité de la préparation.

Pour ces raisons, il parait indispensable d'effectuer les tests d'immunogénicité sur la préparation qui contient la protéine et non sur des peptides de synthèse.

*Les méthodes cellulaires utilisant des protéines et des peptides*

Tangri *et al.,* décrivent une méthode d'évaluation de l'immunogénicité de protéines chez l'homme à partir de cellules mononuclées du sang périphérique (PBMC) de donneurs naïfs, comprenant :

- Etape 1 : la séparation des monocytes et des lymphocytes T CD4+,
- Etape 2 : la préparation de cellules dendritiques (DC) chargées avec la protéine par culture des monocytes pendant 7 jours en présence GM-CSF et d'IL-4, le chargement des DC avec la protéine pendant quelques heures, puis le lavage et la centrifugation des DC. Des monocytes cultivés dans les mêmes conditions, à l'exception de l'étape de chargement qui est effectuée dans un milieu ne contenant pas la protéine, sont utilisées comme contrôle,

- Etape 3 : la stimulation des lymphocytes T CD4+ par co-culture avec des DC chargées avec la protéine (stimulation initiale), puis avec des PBMC chargées avec la protéine ou avec des peptides immunogènes issus de ladite protéine (deux restimulations),
- Etape 4 : la détection de la réponse lymphocytaire T CD4+ spécifique de ladite protéine par un test ELISPOT-IFN-γ comprenant la co-culture des lymphocytes T CD4+ stimulés avec des PBMC chargées avec des peptides issus de la dite protéine, dans des plaques recouvertes d'anticorps anti-IFN-γ, puis la détection immunoenzymatique des cellules productrices d'IFN-γ.

Les épitopes reconnus par les lymphocytes T CD4+ sont identifiés à partir de peptides chevauchants couvrant l'ensemble de la séquence de la protéine à étudier et au moyen de donneurs naïfs d'haplotype HLA II variés. La fréquence d'individus répondeurs est déterminée pour chaque peptide.

Cette méthode est sensible et spécifique étant donné qu'elle permet de comparer l'immunogénicité d'une protéine de type sauvage avec celle de variants dont l'immunogénicité a été diminuée par modification de l'affinité pour les molécules HLA-DR d'épitopes immunodominants de cette protéine. Toutefois, comme l'étape 4 de mise en évidence de la spécificité des lymphocytes T CD4+ se fait au moyen de peptides, elle ne permet pas de détecter la présence éventuelle de molécules contaminantes qui participent à l'immunogénicité des préparations de protéines et de prendre en compte la formulation de la protéine, l'existence de modifications post-traductionnelles, d'altérations éventuelles des acides aminés (telles que l'oxydation) ou des modification introduites dans la protéine telles que la pegylation de la forme galénique de la protéine.

En outre, pour comparer les différents variants, elle présente l'inconvénient majeur d'utiliser des peptides antigéniques correspondant à des épitopes T CD4+ de la protéine à tester, ce qui implique l'identification préalable de ces épitopes T CD4+ à partir de peptides couvrant l'ensemble de la séquence de la protéine à étudier, ce qui est long, fastidieux et couteux.

*Les méthodes cellulaires utilisant uniquement des protéines,*

Une telle méthode d'évaluation de l'immunogénicité a été récemment décrite par Jaber et Baker (Jaber, A. et M. Baker, J. Pharm. Biomed Anal., 2007) et dérive de travaux antérieurs décrits par d'autres (Schlienger et al., Blood, 2000, 96:3490-3498).

Cette méthode est plus simple et rapide que la méthode décrite par *Tangri et al.* En outre elle n'utilise pas de peptides mais uniquement la protéine à analyser. De manière plus précise, la méthode décrite par Jaber, A. et M. Baker comprend l'Etape 1 telle que décrite ci-dessus, l'Etape 2 comprend une étape supplémentaire d'incubation des DC immatures chargées avec la protéine, pendant 24 h en présence d'un agent de maturation (TNF-α) de façon à obtenir des DC matures chargées avec la protéine. L'Etape 3 de stimulation des lymphocytes T CD4+ par co-culture avec des DC matures chargées avec la protéine à analyser ne comprend pas de restimulation. L'Etape 4 de détection de la réponse lymphocytaire T CD4+ spécifique de ladite protéine est effectuée de façon concomitante à l'étape 3 de stimulation des lymphocytes T CD4+ (à la fin de l'Etape 3, *i.e.,* après 6 à 7 jours de co-culture) par un test ELISPOT-IFN-γ ou un test de prolifération lymphocytaire.

Le chargement en protéine des DC est effectué avant l'induction de leur maturation dans la mesure où il a été montré que le chargement des DC immatures avec l'antigène avant d'induire leur maturation était essentiel pour obtenir des DC matures capables d'activer des lymphocytes T CD4+ naïfs de façon efficace (Schlienger et al., Blood, 2000, 96, 3490-3498).

Cette méthode présente plusieurs inconvénients. En effet, les auteurs utilisent comme témoin positif une protéine connue comme étant fortement immunogène (KLH : *Keyhole Limpet Hemocyanin*) et qui sert notamment de référence dans des essais de vaccination. La réponse obtenue contre la KLH est supérieure à celle du bruit de fond (réponse lymphocytaire T CD4+ en présence des DC contrôle), seulement d'un facteur 1,4 dans le test ELISPOT et d'un facteur 2,1 dans le test de prolifération. Ces rapports signal sur bruit de fond sont bas et indiquent que la méthode est peu sensible. Une protéine moins immunogène que la KLH, comme c'est le cas des interférons beta-1a testés dans cette étude, donne en effet naissance à une réponse peu différente du bruit de fond. On peut également noter que la réponse d'une des protéines (RNF2) est même inférieure au bruit de fond ce qui met en en lumière la faible spécificité de cette méthode.

Il apparaît donc que les méthodes non-cellulaires et les méthodes cellulaires utilisant uniquement des peptides souffrent d'inconvénients majeurs qui ne permettent pas d'obtenir de résultats fiables.

Les méthodes cellulaires qui utilisent des protéines combinent l'ensemble des phases de l'activation des lymphocytes T CD4+ et reproduisent l'intégralité du processus d'activation des lymphocytes T CD4+ naïfs humains. Toutefois, seule la méthode mixte qui utilise des protéines et des peptides est sensible et spécifique. La méthode cellulaire qui utilise uniquement des protéines est peu spécifique et peu sensible.

Par conséquent, les méthodes cellulaires d'évaluation de l'immunogénicité des protéines actuellement proposées ne permettent pas d'obtenir des résultats sensibles et spécifiques sans s'affranchir de l'utilisation de peptides qui est coûteuse et peut masquer les effets dus aux modifications accidentelles ou volontaires des séquences naturelles des protéines thérapeutiques. En outre, elles ne permettent pas de détecter la présence éventuelle de molécules contaminantes qui participent à l'immunogénicité des préparations de protéines et elles ne prennent pas en compte la formulation de la protéine ou son procédé de production.

La présente invention a pour but de palier les inconvénients des méthodes de l'art antérieur en offrant un procédé spécifique et sensible d'évaluation de l'immunogénicité des protéines *in vitro* qui utilise la protéine à tester comme seul antigène pour mesurer la réponse lymphocytaire T CD4+ spécifique de cette protéine.

Les inventeurs ont mis en évidence que la mesure de la réponse lymphocytaire T CD4+ spécifique d'une protéine à partir de lymphocytes T CD4+ naïfs nécessite une activation préalable des lymphocytes T CD4+ dans une étape séparée de la détection de la réponse lymphocytaire T CD4+ spécifique de cette protéine. En outre, le choix des cellules présentatrices de l'antigène à tester (protéine) ainsi que la préparation de ces dernières permettent d'améliorer la sensibilité et la spécificité de cette réponse lymphocytaire T CD4+ spécifique.

Le procédé de l'invention mesure l'immunogénicité d'une préparation de protéine en tenant compte de la spécificité pour ladite préparation de protéine, des lymphocytes T CD4+ activés par ladite préparation. Cette mesure directe de l'immunogénicité des protéines respecte l'apprêtement physiologique de la protéine dans la cellule présentatrice et est plus performante.

La sensibilité et la spécificité du procédé de l'invention sont démontrées au Tableau I. On observe des intensités de réponse élevées avec des protéines connues comme étant immunogènes chez l'homme (OVA, KLH et anticorps monoclonal murin, M$\alpha$2,3 (Trémeau et al., FEBS Lett., 1986, 208 (2), 236-240)). En revanche, les intensités de réponse sont très faibles ou nulles avec des protéines non-immunogènes chez l'homme (insuline humaine, anti-trypsine humaine, anti-thrombine humaine). Le procédé selon l'invention a été utilisé avec succès pour comparer l'immunogénicité de différentes protéines thérapeutiques humaines ou chimériques (humaine-murine) et de protéines connues comme étant immunogènes chez l'homme (KLH et un anticorps monoclonal murin, M$\alpha$2,3). Ainsi, le procédé selon l'invention a permis de classer l'immunogénicité relative d'une série de protéines thérapeutiques, par rapport à celle de la KLH et de l'anticorps murin M$\alpha$2,3. Comme le montre les Tableaux II et V, les valeurs d'immunogénicité relative des différentes protéines obtenues selon le procédé de l'invention sont corrélées avec les valeurs obtenues cliniquement (pourcentage d'individus présentant des anticorps spécifiques).

Le procédé selon l'invention permet d'évaluer l'immunogénicité des protéines et notamment de classer l'immunogénicité d'une première protéine par rapport à l'immunogénicité d'au moins une deuxième protéine. L'invention est remarquable en ce qu'elle permet de cribler des protéines candidates et de comparer leur immunogénicité par rapport à une référence avant leur administration chez l'homme. Le procédé selon l'invention permet en particulier de sélectionner une protéine améliorée par évolution dirigée tout en possédant une immunogénicité comparable voire plus faible que la protéine de référence.

Le procédé selon l'invention peut être utilisé pour cribler des protéines thérapeutiques possédant une immunogénicité réduite, neutralisée ou supérieure par rapport à la protéine de référence très immunogène comme par exemple la KLH ou peu immunogène comme l'insuline humaine.

Le procédé selon l'invention peut être mis en oeuvre pour cribler au contraire des protéines vaccins induisant des réponses immunitaires suffisamment efficaces par rapport à la protéine vaccin de référence.

Le procédé selon l'invention peut également être mis en oeuvre pour comparer l'immunogénicité d'une protéine selon son procédé de production, sa formulation, son lot de fabrication.

Le procédé selon l'invention peut aussi être mis en oeuvre pour comparer l'immunogénicité d'une protéine modifiée par rapport à une protéine de référence (non-modifiée) et ainsi évaluer l'effet des modifications introduites dans une protéine sur l'immunogénicité de cette protéine.

La présente invention a pour objet un procédé d'évaluation de l'immunogénicité de protéines, comprenant au moins les étapes suivantes :

a) l'activation de lymphocytes T CD4+ d'au moins un donneur par co-culture de lymphocytes T CD4$^+$ de chaque donneur avec des cellules présentatrices d'antigène autologues chargées avec une protéine à tester, dans au moins dix récipients de culture indépendants (n $\geq$ 10) pour chaque donneur,
b) la mesure de l'activation desdits lymphocytes T CD4+ vis-à-vis de cellules dendritiques immatures autologues chargées avec ladite protéine (valeur Ai avec $1 \leq i \leq n$) et là mesure en parallèle de l'activation desdits lymphocytes T CD4+ vis-à-vis de cellules dendritiques immatures autologues non-chargées (valeur *Bi* avec $1 \leq i \leq n$), pour chacune des *n* cultures de lymphocytes T CD4$^+$ de chaque donneur de l'étape a), et
c) le calcul de la valeur de l'immunogénicité de ladite protéine par comparaison de l'ensemble des valeurs *Ai* avec l'ensemble des valeurs *Bi,* obtenues pour l'ensemble des cultures de lymphocytes T CD4$^+$ des donneurs de l'étape a) (*n* cultures par donneur).

*Définitions*

- On entend par « <u>antigène</u> » toute molécule, notamment une protéine ou un peptide, pouvant être reconnue par le système immunitaire et en particulier par les lymphocytes T CD4+.
- On entend par « <u>cellule présentatrice d'antigène ou CPA</u>», une cellule exprimant une ou plusieurs molécules CMH de classe II (molécules HLA de classe II chez l'homme) et capables de présenter les antigènes aux lymphocytes T CD4+ spécifiques de cet antigène. Comme cellules présentatrices d'antigène, on peut citer notamment les cellules dendritiques (DC), les cellules mononuclées du sang périphérique (PBMC), les monocytes, les macrophages, les lymphocytes B, les lignées lymphoblastoides, et les lignées de cellules humaines ou animales génétiquement modifiées exprimant des molécules CMH de classe II, notamment des molécules HLA II.
- On entend par « <u>protéine</u> » n'importe qu'elle protéine naturelle, synthétique ou recombinante. Ce terme englobe également les protéines modifiées résultant notamment de la modification de la séquence d'acides aminés (mutations par exemple) d'une protéine, de l'introduction de modifications post-traductionnelles (glycosylation par exemple) dans une protéine, de la dénaturation d'une protéine (desorganisation de la structure spatiale sans rupture des liaisons covalentes) et de la complexation ou du couplage (formation d'un conjugué) d'une protéine à un composé organique tel que par exemple une molécule thérapeutique ou un métabolite dérivé de cette molécule.
- On entend par « <u>cellule présentatrice d'antigène chargée par une protéine, chargée avec une protéine ou chargée en protéine</u> », une cellule présentatrice qui a internalisé ladite protéine (endocytose) et la dégradée en peptides (dégradation protéolytique) qui ont été chargés sur des molécules CMH de classe II et sont présentés à la surface de ladite cellule présentatrice sous la forme de complexes CMH II/peptide.
- On entend par « <u>chargement d'une cellule présentatrice en protéine</u>», la co-incubation de ladite cellule présentatrice avec une protéine dans des conditions permettant l'internalisation, la dégradation, le chargement sur des molécules CMH II et la présentation de la dite protéine sous la forme de complexes CMH II/peptide dérivé de ladite protéine, exprimés à la surface de ladite cellule présentatrice. Les cellules présentatrices non-chargées sont préparées en parallèle, en l'absence de la protéine.
- On entend par « <u>lymphocyte T CD4+</u> », un lymphocyte T CD4+ qui a été ou non en contact avec l'antigène.
- On entend par « <u>lymphocyte T CD4+ naïf</u> » un lymphocyte T qui n'a jamais été en contact avec l'antigène.
- On entend par « <u>cellule dendritique ou DC</u> », une cellule présentatrice d'antigène capable, en présentant l'antigène, de stimuler des lymphocytes T CD4+ spécifiques de cet antigène.
- On entend par « <u>cellule dendritique immature ou iDC</u> », une cellule dendritique capable d'internaliser et de dégrader les protéines.
- On entend par « <u>cellule dendritique mature ou mDC</u> », une cellule issue de la maturation d'une iDC par un agent de maturation et capable, en présentant l'antigène, de stimuler des lymphocytes T CD4+ spécifiques de cet antigène même si ces lymphocytes CD4+ sont naïfs. Les mDC sont caractérisées par l'expression à leur surface de molécules de co-stimulation telles que CD83, CD86 et CD40 et d'une quantité importante de complexes CMH II/peptide (CD40+, CD83$^{hi}$, CD86$^{hi}$, CMH classe II$^{hi}$)
- On entend par <u>« agent de maturation</u> », une molécule ou un mélange de molécules capable d'induire la maturation des DC immatures en DC matures.
- On entend par « <u>activation, stimulation ou induction de lymphocytes T CD4+</u> », la stimulation des lymphocytes T CD4+ possédant un récepteur T spécifique de complexes peptide/molécule CMH de classe II présentés à la surface de cellules présentatrices d'antigène. Cette activation se traduit par la prolifération des lymphocytes T CD4+, ou la sécrétion de cytokines telles que l'IL-2, l'IL-4 ou l'IFN-$\gamma$ ou l'apparition de molécules de co-stimulation telles que le CD40L.

Lorsque les lymphocytes T CD4+ sont naïfs, l'activation est optimale lorsque les cellules présentatrices d'antigène sont des cellules dendritiques matures présentant des complexes peptide/molécule CMH de classe II reconnus par le récepteur T desdits lymphocytes T CD4+.

- On entend par « <u>lignée de lymphocytes T</u> » l'ensemble des lymphocytes T contenus dans un récipient de culture indépendant, notamment dans un puits de plaque de culture.
- On entend par « <u>lignée de lymphocytes T spécifique d'une protéine »</u> une lignée de lymphocytes T qui est activée de manière significative par des DC chargées avec la protéine par rapport aux mêmes DC non chargées.
- On entend par « <u>donneur</u> » ou « <u>donneur de lymphocytes T CD4+»</u> un individu, humain ou animal, notamment un mammifère humain ou non-humain dont sont issus les lymphocytes T CD4+ qui sont mis en culture à l'étape a) du procédé de l'invention.
- On entend par « <u>cellules autologues</u> » des cellules issues du même individu, humain ou animal, notamment un mammifère humain ou non-humain.

- On entend par « <u>immunogénicité</u> » d'une protéine sa capacité à induire une réponse immunitaire spécifique, notamment l'induction de l'activation de lymphocytes T CD4+ spécifiques de ladite protéine.
- On entend par « <u>évaluation de l'immunogénicité</u> » la mesure de la réponse lymphocytaire T CD4+ spécifique de la protéine testée. Cette valeur est déterminée par son intensité et par la fréquence d'individus répondeurs.

<u>* L'intensité de la réponse lymphocytaire T CD4+ spécifique</u> est calculée à partir de la différence ou du rapport entre les valeurs $Ai$ et les valeurs $Bi$, selon l'une des méthodes suivantes :

(1) moyenne arithmétique des différences entre les valeurs $Ai$ et $Bi$ (moyenne des ($Ai - Bi$)) ou (moyenne arithmétique des $Ai$) - (moyenne arithmétique des $Bi$) :

$$\frac{(A1-B1) + (A2-B2) + \dots + (An-Bn)}{n} \text{ ou } \frac{(A1 + A2 + \dots + An) - (B1 + B2 + \dots + Bn)}{n}$$

(2) moyenne arithmétique des quotients $Ai$ sur $Bi$ :

$$\frac{(A1/B1 + A2/B2 + \dots + An/Bn)}{n}$$

(3) quotient de la moyenne arithmétique des $Ai$ sur la moyenne arithmétique des $Bi$ :

$$\frac{(A1 + A2 + \dots + An)}{(B1 + B2 + \dots + Bn)}$$

(4) pourcentage de récipients de culture positifs, c'est-à-dire ceux pour lesquels la *valeur A* est au moins le double de la valeur $B$ ($Ai/Bi \geq 2$) avec $Ai \geq S$ où S mesure le bruit de fond de la technique utilisée pour mesurer l'activation des lymphocytes T CD4+ (étape b). $S$ varie en fonction de la spécificité de cette technique. Par exemple, lorsque l'activation est mesurée par ELISPOT, $S$ correspond à 15 spots pour 10 000 lymphocytes T CD4+.

(5) fréquence de lymphocytes T CD4+ spécifiques de la protéine à tester parmi les lymphocytes T CD4+ du donneur. La répartition des lymphocytes T CD4+ spécifiques suit une loi de poisson de paramètre $\lambda$ où $\lambda$ est le nombre moyen de lymphocytes T CD4+ spécifiques par puits. La probabilité P(X=k) qu'il y ait k lymphocytes T CD4+ spécifiques dans un puits est donnée par la formule : P(X=k) = $e^{-\lambda} \lambda^k/k!$. On peut alors calculer la probabilité qu'un puits ne contienne pas de cellules spécifiques en prenant la valeur k=0 cette valeur est P(X=0) = $e^{-\lambda}$. On en déduit la valeur de $\lambda$ = ln(P(X=0)) qui est fonction du nombre de puits négatifs. Le nombre de puits négatifs est en effet une estimation de P(X=0). Ce nombre de puits négatifs se calcule aisément. A partir du nombre de lymphocytes T CD4+ distribuées par puits et de la valeur calculée pour $\lambda$, on remonte au nombre de lymphocytes T CD4+ spécifiques par nombre de lymphocytes T CD4+ distribuées. La fréquence moyenne est notamment exprimée en nombre de lymphocytes T CD4+ spécifiques par millions de lymphocytes T CD4+.

Lorsque l'immunogénicité d'une protéine est mesurée en parallèle sur plusieurs individus, l'immunogénicité de cette protéine pour la collection d'individus testée peut être évaluée directement en faisant les calculs précités ((1), (2), (3) ou (4)) sur la totalité des puits analysés pour l'ensemble des individus de la collection (somme des valeurs $n$ de l'ensemble des individus). Alternativement, les calculs précités peuvent être effectués pour chaque individu, puis la moyenne arithmétique des valeurs obtenues est ensuite calculée.

L'intensité de la réponse T CD4+ est significative (réponse spécifique) lorsque la moyenne des différences ($Ai$-$Bi$) est supérieure ou égale à $S$, la moyenne des quotients $Ai/Bi$ ou le quotient de la moyenne des $Ai$ sur la moyenne des $Bi$ est supérieur ou égal à 2 ou l'un au moins des récipients de culture testés est positif.

<u>* La fréquence d'individus répondeurs.</u> Cette fréquence correspond au pourcentage d'individus d'une collection d'individus testés pour lesquels l'intensité de la réponse T CD4+ est significative.

- On entend par « <u>récipient de culture</u> » tout support adapté à la culture cellulaire, notamment des récipients de culture comprenant des chambres de culture indépendantes comme notamment des plaques de culture contenant des puits de culture indépendants. Il s'agit par exemple de plaques de culture à 6, 24 ou 96 puits.

Conformément au procédé de l'invention les lymphocytes T CD4+ et les cellules présentatrices d'antigène autologues sont issus d'un ou plusieurs individus(donneur(s)) de l'espèce dans laquelle l'immunogénicité de la protéine est analysée. De préférence, il s'agit d'individus humains ou mammifères non-humain, tels que de manière non-limitative, des animaux de laboratoire (souris, rat, lapin, singe), domestiques (chat chien, cobaye, cheval, vache cochon, mouton, chèvre) ou sauvages (félin, pachyderme, ongulé).

Généralement, le donneur est un individu sain qui n'a pas été en contact avec la protéine (individu naïf vis-à-vis de ladite protéine) mais le procédé de l'invention peut également être appliqué à des donneurs sains qui ont été en contact avec la protéine (individus vaccinés avec la protéine) ou à des malades qui n'ont pas été en contact avec la protéine ou qui ont été en contact avec la protéine (protéine thérapeutique, allergène, antigène de tumeur, auto-antigène).

Selon un mode de mise en oeuvre avantageux dudit procédé, les lymphocytes T CD4+ et les cellules présentatrices d'antigène autologues sont issus d'un individu naïf vis-à-vis de ladite protéine. De préférence, il s'agit d'un individu humain, naïf vis-à-vis de ladite protéine.

La protéine à tester est n'importe quelle protéine susceptible d'induire une réponse immunitaire spécifique chez le donneur comme une protéine modifiée ou non-modifiée telle que définie ci-dessus. Il s'agit notamment d'une protéine qui est destinée à être administrée au donneur (antigène vaccinal, protéine ayant une activité thérapeutique qui cible le système immunitaire ou protéine ayant une activité thérapeutique indépendante de la réponse immunitaire) ou est susceptible d'être en contact avec le donneur (allergène, auto-antigène).

Selon un autre mode de mise en oeuvre avantageux dudit procédé, la protéine à tester est une protéine thérapeutique ; il s'agit notamment d'un antigène vaccinal qui induit une réponse immunitaire spécifique d'un agent pathogène ou d'une tumeur ou d'une protéine ayant une activité thérapeutique qui cible le système immunitaire ou indépendante de la réponse immunitaire (anticorps monoclonal humain, chimérique ou humanisé, cytokine....).

La protéine à tester peut être isolée ou incluse dans un mélange, notamment un mélange comprenant des protéines différentes.

Les lymphocytes T CD4+ et les cellules présentatrices d'antigène autologues sont cultivés dans les conditions standard (température, $CO_2$), dans un milieu de culture classique approprié. Les lymphocytes T CD4+ et les cellules présentatrices d'antigène sont notamment isolés à partir de la fraction de cellules mononucléées du sang périphérique (PBMCs) d'au moins un individu de l'espèce dans laquelle l'immunogénicité de la protéine est évaluée, selon les techniques classiques connues de l'Homme du métier, telles que décrites notamment dans Castelli et al., European Journal of Immunology, 2007, 37, 1513-1523. Les PBMCs sont isolées par centrifugation en gradient de densité (gradient de Ficoll) puis mises en culture de façon à séparer les cellules adhérentes (monocytes) et les cellules non-adhérentes (lymphocytes). Les cellules isolées (PBMC, monocytes, lymphocytes, lymphocytes T CD4+, cellules dendritiques) peuvent être mises en culture immédiatement ou congelées dans un milieu standard approprié et mises en culture ultérieurement.

Les lymphocytes T CD4+ sont purifiés à partir des cellules mono-nucléeés non-adhérentes par sélection positive à l'aide d'anticorps anti-CD4, notamment à l'aide d'anticorps anti-CD4 couplés à des billes magnétiques.

Les cellules adhérentes (monocytes) sont utilisées pour préparer des cellules dendritiques (immatures et matures). Typiquement, les cellules dendritiques immatures sont obtenues par culture de cellules mononucléées adhérentes au plastique, pendant 3 à 7 jours, de préférence 5 jours, en présence d'IL-4 (1000 UI/mL) et de GM-CSF (1000 UI/mL). Les cellules dendritiques immatures sont ensuite différenciées en cellules dendritiques matures par culture en présence d'un agent de maturation, par exemple, LPS (lipopolysaccharide d'*E. coli,* TNF-$\alpha$, CD40L ou prostaglandine E2, pendant une durée qui varie en fonction de la concentration d'agent de maturation utilisée et peut être facilement déterminée pour chaque agent de maturation (TNF-$\alpha$ : 20 ng/mL pendant 24 h; LPS: 1 $\mu$g/mL pendant 4 h à 48 h).

Le chargement en protéine des cellules présentatrices d'antigène comprend l'incubation des cellules présentatrices d'antigène avec la protéine (en général 10 à 500 $\mu$g/mL) dans du milieu de culture pour cellules présentatrices d'antigène (monocytes, DC ou PBMC) standard, généralement à 37°C pendant quelques heures. Après le chargement, les cellules présentatrices sont généralement lavées avant d'être remises en culture.

Le chargement en protéine des cellules dendritiques peut être effectué avant l'induction de leur maturation ou simultanément.

Lorsque le chargement en protéine des cellules dendritiques est effectué avant l'induction de leur maturation, les cellules dendritiques immatures sont co-incubées avec la protéine pendant au moins 4 h, ensuite elles sont généralement lavées avant d'être mises en culture en présence de l'agent de maturation pendant le temps nécessaire à leur maturation qui est défini en fonction de la nature et de la concentration d'agent de maturation, comme précisé ci-dessus.

Lorsque le chargement en protéine des cellules dendritiques est effectué simultanément à l'induction de leur maturation, les cellules dendritiques immatures sont cultivées avec la protéine et l'agent de maturation pendant le temps nécessaire à leur maturation.

Selon un autre mode de mise en oeuvre avantageux dudit procédé, les cellules présentatrices d'antigène chargées en protéine à tester (étape a)) sont des cellules dendritiques matures chargées avec ladite protéine.

Selon une première disposition avantageuse de ce mode de réalisation, lesdites cellules dendritiques matures ont été obtenues par maturation de cellules dendritiques immatures en présence de LPS.

Selon une seconde disposition avantageuse de ce mode de réalisation, les cellules dendritiques matures chargées avec la protéine sont obtenues à partir de cellules dendritiques immatures qui ont été chargées avec la protéine et maturées simultanément avec l'agent de maturation. Les cellules dendritiques immatures ont été incubées simultanément avec la protéine et l'agent de maturation pendant au moins 4 heures, de préférence pendant 4 heures à 48 heures, de manière préférée pendant 24 h.

Comme indiqué précédemment, les méthodes de l'art antérieur mettent en oeuvre le chargement en protéine des DC avant l'induction de leur maturation, dans la mesure où il a été montré que le chargement des DC immatures avec l'antigène avant d'induire leur maturation était essentiel pour obtenir des DC matures capables d'activer des lymphocytes T CD4+ naïfs de façon efficace (Schlienger et al., Blood, 2000, 96, 3490-3498).

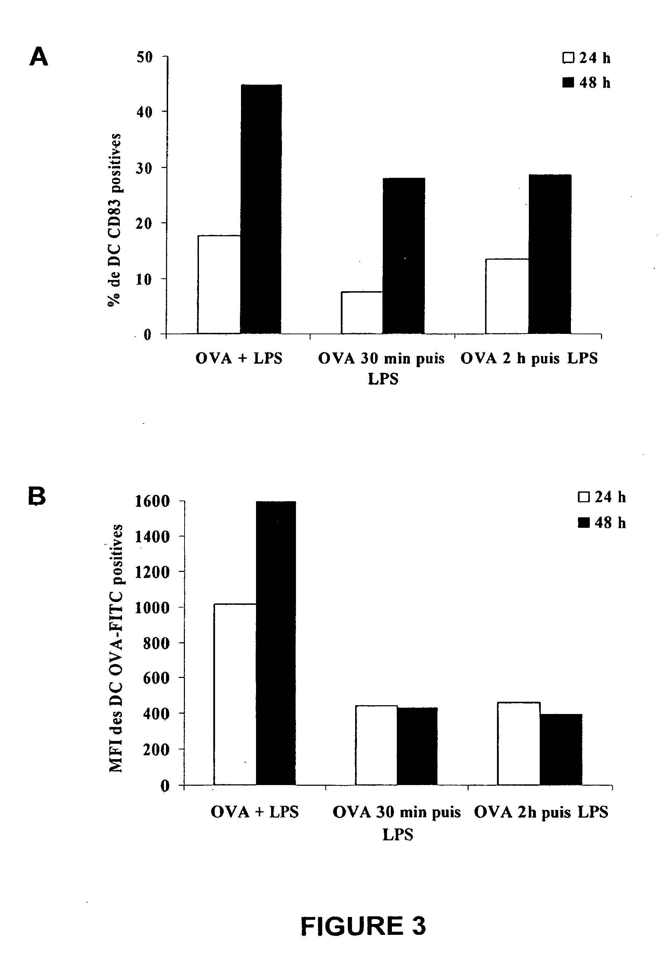

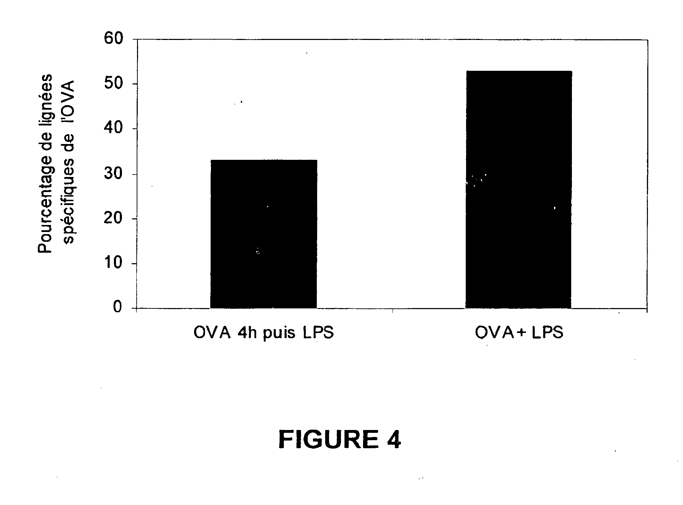

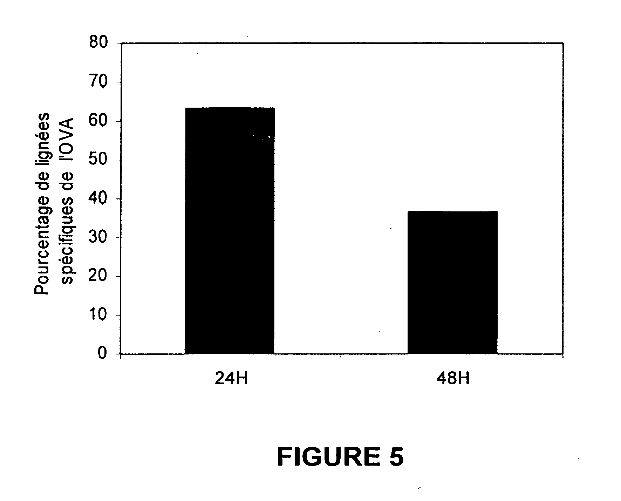

De façon étonnante les inventeurs se sont rendu compte que le chargement des DC immatures avec la protéine et la maturation simultanée des DC immatures permettent d'activer efficacement et rapidement la réponse des lymphocytes T CD4+ tout en limitant la perte de cellules et leur risque de contamination. Les inventeurs ont montré de façon surprenante que les DC peuvent endocytoser une protéine de façon continue même si elles ont initié leur maturation, alors que l'incubation des DC avec la protéine avant leur maturation limite la capture de la protéine (figure 3B). En outre, la mise en contact simultanée des DC et de la protéine ne perturbe pas leur maturation, alors que la maturation est moins efficace lorsque la protéine et l'agent de maturation sont ajoutés séparément (figure 3A). Ces étapes de chargement des DC immatures avec la protéine et de maturation simultanée permettent d'obtenir de façon performante et optimale des DC chargées avec la protéine d'intérêt et rendues matures pour l'induction de lymphocytes T CD4+ spécifiques. Cette méthode d'activation performante des lymphocytes T par les protéines permet de mesurer efficacement la spécificité des lignées de lymphocytes T induites en présence de protéines qui est utilisée pour évaluer l'immunogénicité des protéines (Figures 4 et 5).

Conformément au procédé de l'invention, chaque récipient de culture de l'étape a) comprend au moins $10^5$ lymphocytes T CD4+, de préférence $10^5$ à $2.10^5$ lymphocytes T CD4+. Le nombre de cellules présentatrices d'antigène est inférieur à celui des lymphocytes T CD4+ d'au moins un facteur 10, de préférence d'un facteur 10 à 20 ($5.10^3$ à $2.10^4$ cellules présentatrice d'antigène). Les co-cultures sont réalisées dans au moins 10 récipients de culture indépendants; de préférence elles sont réalisées dans 15 récipients indépendants, de manière préférée elles sont réalisées dans 20 récipients indépendants. Les co-cultures peuvent notamment être réalisées dans les puits d'une plaque de culture à 96 puits. Les lymphocytes T CD4+ et les cellules présentatrices d'antigène sont co-cultivés pendant au moins 5 jours.

Selon un autre mode de mise en oeuvre avantageux dudit procédé, l'étape a) comprend au moins une restimulation des lymphocytes T CD4+ par l'ajout à la co-culture, de cellules présentatrices d'antigène autologues chargées avec la protéine à tester telles que définies précédemment. Selon une disposition avantageuse de ce mode de réalisation, les restimulations sont effectuées tous les 5 à 7 jours, après au moins 5 jours de co-culture, de préférence après 5 à 7 jours de co-culture. De préférence, l'étape a) comprend deux restimulations à 5 à 7 jours d'intervalle, la première étant réalisée après 5 à 7 jours de co-culture.

Conformément au procédé de l'invention, l'étape b) comprend l'analyse de la spécificité des lignées de lymphocytes T CD4+ activés obtenues à l'étape a). Les étapes a) et b) sont réalisées de façon séparée. La mesure de l'activation des lymphocytes T CD4+ est réalisée par mise en culture des lymphocytes T CD4+ activés obtenus à la fin de l'étape a) avec une nouvelle préparation de cellules présentatrices d'antigène (DC immatures) autologues chargées avec la protéine à tester..

L'activation des lymphocytes T CD4+ est mesurée en utilisant comme cellules présentatrices d'antigène, des cellules dendritiques immatures chargées en protéine à tester, préparées comme décrit ci-dessus. Des cellules dendritiques immatures incubées en l'absence de protéine sont utilisées comme contrôle de la spécificité des lignées de lymphocytes T CD4+ activés obtenues à l'étape a). La mesure de l'activation des lymphocytes T CD4+ comprend la mesure de la prolifération, de la production de cytokine(s) spécifique(s) (IL-2, IL-4 ou IFN-γ) ou de l'expression de marqueurs d'activation des lymphocytes T CD4+ (CD40L) par toute technique classique connue de l'Homme du métier. La prolifération des lymphocytes T CD4+ peut notamment être évaluée par mesure de l'incorporation de thymidine tritiée ou du marquage par le CFSE (5- et 6- carboxyfluoresceine diacetate succimidyl ester). La production de cytokine(s) spécifique(s) et l'expression des marqueurs d'activation peuvent être évaluées par l'analyse des transcrits (RT-PCR) ou l'immunodétection de la protéine correspondante sous forme intracellulaire ou extracellulaire (cytokines) ou exprimée à la membrane des lymphocytes T CD4+ (marqueurs d'activation)), notamment par ELISA, RIA, ELISPOT, FACS, comme décrit précédemment (Castelli et al., European Journal of Immunology, 2007, 37, 1513-1523; Castelli et al., European Journal of Immunology, 2008, 38, 1-11).

Selon un autre mode de mise en oeuvre avantageux dudit procédé, l'activation des lymphocytes T CD4+ (étape b) est mesurée par un test de prolifération lymphocytaire, un test de marquage de cytokine(s) intracellulaire(s) ou un test ELISPOT. Selon une disposition avantageuse de mode de réalisation, l'étape b) est réalisée par un test ELISPOT, de préférence un test ELISPOT- IFN-γ.

Lors de la mise en oeuvre de l'étape b), les inventeurs ont mis en évidence différents types de profils de réponse des lymphocytes T activés (lymphocytes T CD4+ issus de l'étape a)) et l'importance de ces différences de réponse dans la mesure de l'immunogénicité d'une protéine (étape c).

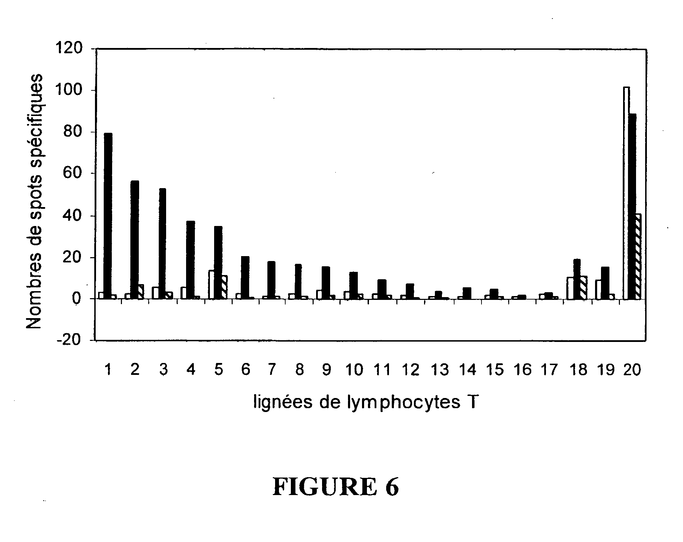

En effet, les inventeurs ont démontré (figure 6) que les lignées de lymphocytes T CD4+ activés issues d'un individu présentent trois types de profils de réponse vis-à-vis de cellules dendritiques immatures chargées en protéine à tester et de cellules dendritiques immatures non-chargées (sans protéine):

1) réponse positive vis-à-vis de la protéine et réponse négative en l'absence de protéine; il s'agit de lignées de lymphocytes T CD4+ spécifiques de la protéine à tester (lignées 1 à 9 de la figure 6),
2) réponse négative en présence ou en l'absence de la protéine; il s'agit de lignées de lymphocytes T CD4+ non-spécifiques de la protéine à tester (lignées 10 à 17 de la figure 6), et
3) réponse positive en présence ou en l'absence de la protéine; il s'agit de lignées de lymphocytes T CD4+ non-spécifiques de la protéine à tester (lignées 18 à 20 de la figure 6).

L'évaluation de l'immunogénicité (étape c)) tient compte de l'existence de ces trois profils de réponse. L'immunogénicité peut être exprimée par l'intensité de la réponse et la fréquence d'individus répondeurs, déterminées comme précisé ci-dessus. Elle permet de distinguer au moins quatre types de protéines : des proteines ayant un intensité forte et une fréquence de répondeurs élevés, des protéines ayant une intensité forte mais chez un nombre réduit d'individu, des protéines ayant une intensité faible avec une fréquence élevée d'individus et des protéines ayant une intensité faible et une fréquence faible de répondeurs. De préférence, la valeur de l'immunogénicité de la protéine à tester est comparée à celle d'au moins une protéine standard dont l'immunogénicité est connue, de façon à classer la protéine à tester par rapport à une échelle de valeurs d'immunogénicité obtenues pour lesdites protéines standard.

Selon une disposition particulière des modes de réalisation précédents, ledit procédé comprend les différentes étapes suivantes :

* <u>étape a)</u> :

-   l'obtention de cellules dendritiques immatures (iDC) et de lymphocytes T CD4+ à partir d'un échantillon biologique d'un même donneur,
-   l'obtention de DC matures chargées en protéine par chargement des iDC avec la protéine à tester et la maturation simultanée des iDC avec un agent de maturation, de préférence le LPS, pendant au moins 4 h, de préférence 24 h,
-   la co-culture desdits lymphocytes T CD4+ avec lesdites DC matures chargées en protéine, dans au moins 10 puits indépendants d'une microplaque à 96 puits, de préférence 15 puits, pendant 5 à 7 jours,
-   l'addition à la dite co-culture de DC matures chargées en protéine obtenues comme décrit ci-dessus, au moins une fois et de préférence deux fois à 5 à 7 jours d'intervalle.

* <u>étape b)</u> : la mesure de l'activation desdits lymphocytes T CD4+ vis-à-vis de cellules dendritiques immatures autologues chargées avec ladite protéine (valeur $Ai$ avec $1 \leq i \leq n$) et la mesure en parallèle de la réponse desdits lymphocytes T CD4+ vis-à-vis de cellules dendritiques immatures autologues non-chargées (valeur $Bi$ avec $1 \leq i \leq n$), pour chacune des $n$ cultures de lymphocytes T CD4$^+$ de l'étape a) ; de préférence l'activation est mesurée par un test ELISPOT-IFNγ,
* <u>étape c)</u> : le calcul de l'intensité de la réponse, comme précisé ci-dessus.

De préférence, l'obtention de iDC et de lymphocytes T CD4+ à l'étape a) comprend les étapes suivantes :

-   l'isolement de cellules mononuclées (PBMC) à partir d'un échantillon de sang périphérique d'un donneur, notamment par centrifugation sur un gradient de Ficoll,
-   la mise en culture des cellules mononuclées sur un support en plastique de manière à isoler les cellules adhérentes,
-   la purification des lymphocytes T CD4+ à partir des cellules non adhérentes, notamment par sélection positive à l'aide d'anticorps anti-CD4+ couplés à un support tel que des billes magnétiques, et
-   la génération de cellules dendritiques immatures par culture des cellules adhérentes en présence de GM-CSF et d'IL4 pendant 3 à 7 jours, de préférence 5 jours.

Selon un autre mode de mise en oeuvre avantageux dudit procédé, les étapes a) à c) sont réalisées en parallèle sur des cultures issues d'une collection d'individus différents et l'étape c) comprend le calcul de l'intensité de la réponse et de la fréquence de répondeurs dans la collection d'individus testés.

Selon une disposition avantageuse de ce mode de réalisation, la collection d'individus testés est représentative d'une population d'individus dans laquelle l'immunogénicité de la protéine est analysée. Pour ce faire, la collection d'individus est sélectionnée de sorte que les fréquences des allèles HLA-DR dans la collection d'individus soient proches de celles rencontrées dans la population d'individus étudiée. La population d'individus correspond par exemple à celle à laquelle la protéine est destinée à être administrée. Alternativement, il s'agit d'une population à risque, notamment et sans que cela soit exclusif une population présentant une pathologie chronique, une population pour laquelle on a observé une proportion de réponse immunitaire contre la protéine supérieure à la normale ou une population ayant des anticorps contre la protéine.

Selon un autre mode de mise en oeuvre avantageux dudit procédé, les étapes a) à c) sont réalisées successivement ou simultanément avec différentes protéines à tester puis les valeurs d'immunogénicité obtenues à l'étape c) sont comparées entre elles.

Ce mode de mise en oeuvre permet de comparer l'immunogénicité de plusieurs protéines. Il permet notamment de comparer l'immunogénicité d'au moins une protéine à tester avec celle d'une protéine de référence. Il permet également de comparer l'immunogénicité de protéines similaires, notamment de variants d'une protéine, améliorés par évolution dirigée ou de protéines modifiées telles que définies ci-dessus. Ainsi, le procédé selon l'invention permet de cribler et de sélectionner des protéines candidates possédant l'immunogénicité désirée et de comparer leur immunogénicité par rapport à une référence avant leur administration chez l'homme. Il s'agit notamment de protéines thérapeutiques dont l'activité est améliorée par évolution dirigée tout en possédant une immunogénicité comparable voire plus faible que la protéine de référence. Il s'agit également de protéines thérapeutiques possédant une immunogénicité réduite, neutralisée ou supérieure par rapport à la protéine de référence très immunogène comme par exemple la KLH ou peu immunogène comme l'insuline humaine. Il s'agit encore de protéines vaccins induisant des réponses immunitaires suffisamment efficaces par rapport à la protéine vaccin de référence.

Le procédé de l'invention permet également de comparer l'immunogénicité d'une même protéine à différentes étapes de son processus de production, selon son lot de fabrication, selon sa formulation ou son procédé de production. Ainsi, le procédé selon l'invention permet de contrôler l'immunogénicité d'une protéine thérapeutique au cours de sa production et de sa formulation.

Le procédé de l'invention permet encore de comparer l'immunogénicité d'une protéine thérapeutique dans différentes populations d'individus. Il s'agit notamment de comparer l'immunogénicité d'une protéine thérapeutique dans une population normale et dans une population à risque.

Ainsi, le procédé de l'invention est utile pour évaluer l'immunogénicité d'une protéine thérapeutique, que ce soit au cours du développement d'un médicament : stade pré-clinique (sélection d'une protéine candidate) et clinique (production, formulation), ou tout au long de la vie de ce médicament, après son autorisation de mise sur le marché (études spécifiques sur des populations à risque ou de nouvelles populations cibles candidates au traitement/vaccination).

Ainsi, au cours de la phase de Recherche et Développement et notamment lors des phases pré-cliniques, le procédé permet le criblage immunogénique des futurs candidats prédisposés aux études cliniques. Ces futurs candidats pourront par exemple être sélectionnés par comparaison de différents variants d'un même produit à une protéine de référence qui peut être une protéine dont l'immunogénicité est connue ou une protéine déjà utilisée en clinique. Ce procédé est également un outil intéressant pour évaluer l'immunogénicité des biosimilaires, en comparaison avec le produit référent.

Au cours des phases cliniques, ce procédé permet de définir la signature immunogénique d'un produit thérapeutique contenant une protéine thérapeutique, soit en d'autre terme, les caractéristiques immunologiques intrinsèques du dit produit. Ce procédé permet ainsi une étude sur un échantillon d'individus représentatif de la population, afin de définir de façon significative le niveau de risque d'immunogénicité du produit. Il deviendra alors possible d'associer un risque d'immunogénicité dudit produit à un phénotype HLA particulier, si ce risque existe, permettant ainsi l'identification des populations à risque. Enfin, les effets de toutes modifications de la protéine thérapeutique ou de son procédé de fabrication sur l'immunogénicité de cette protéine thérapeutique pourront également être suivis.

L'évolution de la signature immunogénique d'un produit thérapeutique pourra être évaluée après la mise sur le marché de ce dit produit. On entend par évolution de la signature immunogénique, l'étude du risque d'immunogénicité d'un produit lorsque son procédé de fabrication est modifié, ou lorsque son utilisation est élargie à une pathologie autre que celle pour laquelle il a été conçu.

La mise en oeuvre du procédé de l'invention utilise, sauf indication contraire, des méthodes classiques d'immunologie, de culture cellulaire, de biologie cellulaire, de biologie moléculaire et d'ADN recombinant qui sont connues de l'homme du métier. Ces techniques sont décrites en détail dans la littérature, se référer par exemple à: Current Protocols in Molecular Biology (Frederick M. AUSUBEL, 2000, Wiley and Sons Inc, Library of Congress, USA); Current Protocols in Immunology (John E. Coligan et al., 2008, Wiley and Sons Inc, Library of Congress, USA), Molecular Cloning: A Laboratory Manual, Third Edition, (Sambrook et al, 2001, Cold Spring Harbor, New York: Cold Spring Harbor Laboratory Press); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols. 154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986).

D'autres avantages et caractéristiques de l'invention apparaîtront dans les exemples de mise en oeuvre du procédé de l'invention qui suivent. Ces exemples qui montrent que le procédé de l'invention permet d'évaluer l'immunogénicité *in vitro* de protéines de manière spécifique et sensible font référence aux dessins annexés dans lesquels :

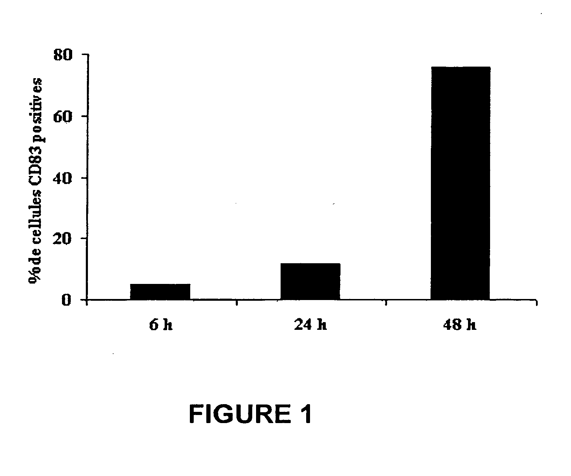

- la figure 1 représente la cinétique de maturation des cellules dendritiques (DC). Des cellules. dendritiques immatures différenciées à partir de monocytes humains ont été cultivées en présence de lipopolysaccharide (LPS) pendant 6, 24 ou 48 heures. La maturation des DC a ensuite été évaluée par l'expression du marqueur CD83 en cytométrie de flux.

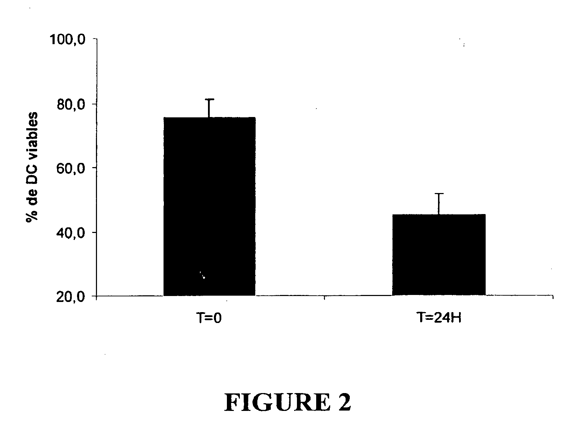

- la figure 2 représente la cinétique de viabilité des DC après induction de la maturation par le LPS. La viabilité des cellules dendritiques a été évaluée p ar numération des cellules après coloration au bleu trypan. Les valeurs correspondent à la moyenne des résultats de trois donneurs.
- la figure 3 représente (A) la cinétique de maturation des DC dans différentes conditions de chargement en protéine et de maturation évaluée en cytométrie de flux, par l'expression du marqueur CD83 par les cellules dendritiques (DC-SIGN positives) et (B) la cinétique de capture de la protéine (OVA-FITC) par les DC selon différentes conditions de chargement en protéine et de maturation, évaluée en cytométrie de flux, par l'intensité de fluorescence moyenne (MFI) de l'OVA-FITC des cellules dendritiques (DC-SIGN positives).
- la figure 4 représente l'effet des différents modes de chargement en OVA et de maturation des DC sur le nombre de lignées de lymphocytes T CD4+ spécifiques de l'OVA. Des DC autologues ont été préparées soit en incubant les DC immatures simultanément avec l'OVA et le LPS pendant 24 h, soit en incubant les DC immatures avec l'OVA pendant 4 h puis avec le LPS pendant 20 h, après une étape intermédiaire de lavage des DC entre le chargement et la maturation. Les DC matures autologues chargées en OVA ainsi obtenues ont servi à induire des lymphocytes T CD4+ spécifiques de l'OVA chez un donneur. La spécificité des lignées de lymphocytes T CD4+ induites par l'OVA a été évaluée par ELISPOT-IFN-$\gamma$.
- la figure 5 représente l'effet du temps de chargement en OVA et de maturation des DC sur le nombre de lignées de lymphocytes T CD4+ spécifiques de l'OVA. Des DC autologues ont été préparées en incubant les DC immatures simultanément avec l'OVA et le LPS pendant 24 h ou 48 h. Les DC matures autologues chargées en OVA ainsi obtenues ont servi à induire des lymphocytes T CD4+ spécifiques de l'OVA chez un donneur. La spécificité des lignées de lymphocytes T CD4+ induites par l'OVA a été évaluée par ELISPOT-IFN-$\gamma$.
- la figure 6 illustre l'hétérogénéité des réponses de lignées de lymphocytes T CD4+ dirigées contre l'OVA évaluées par ELISPOT-IFN-$\gamma$. Des lignées de lymphocytes T CD4+ dirigées contre l'OVA ont été obtenues à partir de PBMC d'un seul donneur et leur spécificité a été testée par ELISPOT-IFN-$\gamma$. Chaque lignée a été testée en triplicat, en ELISPOT-IFN-$\gamma$ vis-à vis de DC immatures autologues chargées avec l'OVA (barres noires), avec la KLH (barres hachurées) et non chargées (barres blanches). Les valeurs correspondent au nombre de spots par puits.

**Exemple 1 : Cinétique de maturation et de viabilité des cellules dendritiques**

**1) Matériel et méthodes**

a) Préparation de cellules dendritiques

Les cellules mononuclées du sang périphérique (PBMC) de donneurs sains ont été isolées par centrifugation en gradient de densité (Ficoll-Hyperpaque gradient, SIGMA-ALDRICH). Le génotype HLA-DR et HLA-DP a été déterminé, soit par séquençage d'HLA-DRB à l'aide d'amorces de PCR appropriées (APPLIED BIOSYSTEMS), soit par SSP à l'aide du kit de typage OLERUP SSP™ HLA-DPB1 et HLA-DRB1 (OLERUP SSP AB).

Les PBMCs ont été mises en culture dans du milieu AIM-V (INVITROGEN) et incubées dans des flasques, à l'étuve à 37°C en présence de 5 % $CO_2$. Après une nuit d'incubation, les cellules non adhérentes (contenant les lymphocytes T CD4+) ont été récupérées. Les cellules mononuclées adhérentes au plastique ont été différenciées en DC immatures (iDC) par 5 jours de culture dans du milieu AIM V (INVITROGEN) supplémenté par 1000 U/ml de rh-Gm-CSF (R&D SYSTEMS) et 1000 U/ml de rh-IL4 (R&D SYSTEMS), dénommé ci-après milieu AIM-V complet. Les DC immatures ont ensuite été différenciées en cellules dendritiques matures par une incubation supplémentaire dans du milieu AIM-V complet supplémenté par 1 $\mu$g/ml de LPS.

b) Cytométrie de flux

Les DC ont été lavées deux fois avec du tampon pour FACS (PBS plus 2 % FCS) et un double-marquage de surface a été réalisé avec un anticorps anti-DC-SIGN-FITC (BD BIOSCIENCES) et un anticorps anti-CD83-PE (BD BIOSCIENCES), pendant 20 min à 4 °C. Après deux lavages avec du tampon pour FACS, les cellules ont été fixées à l'aide d'une solution de paraformaldehyde (2 % ; SIGMA) pendant 20 min à 4°C. Après deux lavages avec du tampon pour FACS, les cellules ont été remises en suspension dans du tampon pour FACS et analysées en cytométrie de flux (FACSCalibur, BD Bioscience).

c) Analyse de la viabilité cellulaire

Un échantillon de DC a été prélevé de la culture, mélangé à une solution de bleu trypan qui permet de colorer les cellules mortes. Les DC viables (non-colorées) et non-viables (colorées en bleu) ont ensuite été comptées au microscope optique, à l'aide d'un hémacytomètre (cellule de Malassez) et le pourcentage de DC viables a été déterminé.

2) Résultats

a) Cinétique de maturation des DC

Le LPS (lipopolysaccharide) est un composant de la paroi cellulaire d'*Escherichia coli* utilisé pour induire la maturation des DC. Les DC (matures et immatures) sont détectées par l'expression du marqueur DC-SIGN. En revanche, contrairement aux DC immatures, les DC matures expriment un marqueur spécifique qui est le CD83. Il est donc possible de suivre la cinétique de maturation des DC en cytométrie de flux, en évaluant le pourcentage de cellules DC-SIGN positives qui expriment le marqueur CD83.

La cinétique de maturation des DC a été évaluée par l'analyse en cytométrie de flux du marquage de CD83, 6h, 24h et 48h après l'ajout de LPS. L'expérience décrite à la figure 1 montre que la maturation des DC est initiée à 24 h et est optimale à 48 h.

b) Viabilité des DC

Les DC immatures générées à partir de monocytes issus du sang périphérique de donneurs sains, comme précisé ci-dessus, ont été cultivées à 37°C pendant 24 h dans du milieu complet contenant 1 μg/ml de LPS, pour induire leur maturation.

La cinétique de viabilité des DC de trois donneurs à 24 h après l'ajout de LPS, a été évaluée après coloration au bleu trypan. La figure 2 montre que la viabilité des PC diminue fortement au bout de 24 h de culture en présence de LPS.

**Exemple 2 : Chargement en protéine et maturation des DC**

**1) Matériel et méthodes**

La protéine qui a été utilisée est l'ovalbumine d'oeuf de poule (OVA) marquée par le FITC. Les DC immatures ont été préparées et cultivées dans du milieu AIM-V complet, comme décrit à l'exemple 1. L'OVA marquée (150 μg/ml) et le LPS (1 μg/ml) ont été ajoutés à la culture de DC immatures, simultanément ou successivement. Dans le cas où l'ajout est successif, l'OVA marquée a été ajoutée à la culture pendant 30 min ou 2 h puis les cellules ont été lavées avant d'ajouter le LPS. Les DC chargées avec l'OVA marquée ont ensuite été cultivées en présence de LPS pendant une durée totale de 24 h ou 48 h. La maturation des DC a été analysée en cytométrie de flux par double-marquage avec un anticorps anti-DC-SIGN et un anticorps anti-CD83 fluorescents, comme décrit à l'exemple 1. L'intemalisation de la protéine par les DC a été analysée en cytométrie de flux sur des cellules fixées marquées en surface avec un anticorps anti-DC-SIGN fluorescent, comme décrit à l'exemple 1.

**2) Résultats**

La capacité des cellules dendritiques à internaliser une protéine (Figure 3A) et leur maturation (Figure 3B) ont été étudiées en fonction de différentes conditions de mise en contact des cellules avec une protéine et de maturation par le LPS.

Trois conditions ont été testées :

- OVA+LPS : la protéine et le LPS sont mis ensemble en contact avec les DC immatures pendant 24 h ou 48 h,

- OVA 30 min puis LPS : la protéine est mise en contact pendant 30 min avec les DC immatures, puis les cellules sont lavées et incubées avec le LPS pendant la durée restante (23h30 ou 47h30), et
- OVA 2 heures puis LPS : la protéine est mise en contact pendant 2 heures avec les DC immatures, puis les cellules sont lavées et incubées avec le LPS pendant la durée restante (22 h ou 46 h).

On observe (Figure 3A) que la mise en contact simultanée des DC et de la protéine ne perturbe pas la maturation des DC alors que la maturation est moins efficace à 24 h et 48 h quand l'antigène est incubé seul avec les DC qui sont ensuite lavées et remises en culture avec le LPS.

De plus on observe (Figure 3B) que la mise en contact simultanée des DC et de la protéine ne perturbe pas non plus le chargement de la protéine par les DC. Il est plus efficace dans cette condition que lorsqu'on sépare la phase de capture de la protéine et de celle de maturation par le LPS.

Ces résultats montrent donc que la maturation et la capture de la protéine sont plus efficaces lorsque la protéine et le LPS sont ajoutés ensemble à la culture que s'ils sont ajoutés séparément.

**Exemple 3 : Comparaison de la capacité à induire des lignées de lymphocytes T CD4+ spécifiques d'une protéine selon le mode de chargement et de maturation des DC**

**1) Matériel et méthodes**

a) Obtention de lignées de lymphocytes T CD4+ spécifiques de la protéine

Les cellules dendritiques immatures ont été obtenues à partir des cellules mononuclées adhérentes au plastique cultivées pendant 5 jours en présence d'IL-4 et de Gm-CSF, comme décrit à l'exemple 1. La protéine (10 à 500 $\mu$g/ml) et le LPS (1 $\mu$g/ml) ont été incubées avec les DC immatures, simultanément ou successivement. Dans le cas où l'incubation est successive, les DC immatures et la protéine sont co-incubées dans du milieu de culture pendant 4 h à 37 °C puis les DC sont lavées avant d'être incubées avec le LPS dans du milieu de culture, pendant 20 h ou 44 h à 37°C. Dans le cas où l'incubation est simultanée les DC immatures, l'OVA et le LPS sont co-incubés dans du milieu de culture, pendant 24 h ou 48 h à 37°C.

Les lymphocytes T CD4+ ont été purifiés à partir des cellules mononuclées non adhérentes du même donneur, au moyen de billes magnétiques sur lesquelles sont fixés des anticorps anti-CD4 (MILTENYI BIOTECH). Leur pureté a été vérifiée en cytométrie de flux. 15 puits de plaques de culture à 96 puits ont été ensemencés avec 100 000 lymphocytes T CD4+ et 10 000 DC matures chargées avec la protéine, préparées dans chacune des deux conditions précisées ci-dessus, en milieu IMDM (INVITROGEN) contenant 10 % de sérum humain du groupe AB (sérum AB), de la glutamine (24 mM), de l'asparagine (55 mM), de l'arginine (150 mM), (SIGMA), 50U/mL de pénicilline et 50 $\mu$g/mL de streptomycine (INVITROGEN), dénommé ci-après milieu IMDM complet, supplémenté en IL-6 (1000 U/mL; R&D SYSTEMS) et en IL-12 (10 ng/mL; R&D SYSTEMS). Après une semaine et pendant deux semaines supplémentaires, les lymphocytes T CD4+ ont été restimulés par les DC matures chargées avec la protéine, préparées dans les mêmes conditions que pour la stimulation initiale, et cultivés en milieu IMDM complet supplémenté en IL-2 (10 U/mL; R&D SYSTEMS) et en IL-7 (5 ng/mL; R&D SYSTEMS). La spécificité des lignées de lymphocytes T CD4+ ainsi obtenues a été testée par ELISPOT-IFN-$\gamma$. b) ELISPOT-IFN-$\gamma$

Un anticorps monoclonal anti-IFN-$\gamma$ (1-D1K, MABTECH, Stockholm, Suède) dilué à 2,5 $\mu$g/mL dans du PBS (INVITROGEN) a été adsorbé sur des plaques multiscreen HA (MILLIPORE) pendant 1 heure à 37°C. Les plaques ont ensuite été saturées avec du milieu IMDM complet pendant 1 h

Des DC immatures autologues chargées avec la protéine ont été obtenues par co-incubation de la protéine (10 à 500 $\mu$g/ml) avec des DC immatures autologues préparées comme décrit à l'exemple 1, à 37°C pendant 4 h à 24 h dans le milieu AIM-V, puis lavage des DC dans du PBS 1X et remise en suspension des DC en milieu IMDM complet.

Chaque lignée de lymphocytes T CD4+ (5000 à 20000 cellules par puits) a été mise en culture avec des DC immatures autologues (5000 à 20000 cellules par puits) préalablement chargées avec la protéine ou non chargées avec la protéine. Après 16 à 24 heures d'incubation à 37°C, l'IFN-$\gamma$ sécrété est mis en évidence par l'addition successive d'un anticorps anti-IFN-$\gamma$ marqué à la biotine (7-B6-1; MABTECH) (0,25 $\mu$g/mL), de streptavidine- phosphatase (SIGMA) et d'un substrat précipitant NBT/BCIP (SIGMA). La présence de cellules sécrétrices d'IFN-$\gamma$ se traduit par l'apparition au fond du puits de taches (ou spots), chaque spot correspondant à une cellule sécrétrice d'IFN-$\gamma$. Le nombre de spots par puits est évalué par un lecteur ELISPOT (AID). Les lignées spécifiques produisent au moins deux fois plus de spots en présence de DC chargée avec la protéine qu'en présence de DC non chargées avec un minimum de 15 spots/10 000 lymphocytes T CD4+.

**2) Résultats**

L'influence du mode de chargement et de maturation sur la capacité des DC à induire des lymphocytes T CD4+ spécifiques d'une protéine (OVA) a été testée (Figure 4).

Des lignées de lymphocytes T CD4+ spécifiques de l'OVA ont été induites *in vitro* par des stimulations hebdomadaires (une stimulation initiale suivie de trois restimulations) avec des DC préparées de deux manières différentes :

- les DC immatures sont mises en contact avec la protéine (OVA) et du LPS pendant 24 h (OVA+LPS),
- les DC immatures sont mises au contact avec l'OVA pendant 4 h, lavées et incubées avec le LPS pendant 20 h.

Les résultats présentés à la figure 4 montrent que le chargement des DC en OVA et la maturation simultanée produisent plus de lignées spécifiques de l'OVA que lorsque le chargement et la maturation sont effectués l'un après l'autre.

**Exemple 4 : Comparaison de la capacité à induire des lignées de lymphocytes T CD4+ spécifiques d'une protéine en fonction du temps de chargement et de maturation des DC.**

**1) Matériel et méthodes**

Le protocole d'induction et d'évaluation de la spécificité des lignées de lymphocytes T CD4+ est le même que celui décrit à l'exemple 3.

**2) Résultats**

De manière à optimiser le temps de chargement et de maturation, des lignées de lymphocytes T CD4+ spécifiques de l'OVA ont été induites *in vitro* par stimulations hebdomadaires avec des DC préalablement chargées en OVA et maturées simultanément par le LPS pendant 24 h ou 48 h. Les résultats obtenus montrent que le nombre de lignées de lymphocytes T CD4+ spécifiques de l'OVA est supérieur à 24 h qu'à 48 h (Figure 5).

Cette observation confirme que les conditions optimales de chargement et de maturation des DC pour induire des lignées spécifiques d'une protéine est un compromis entre d'une part le chargement de la protéine et de la maturation qui l'un et l'autre augmentent avec les temps d'incubation (Figure 1 et Figure 3) et d'autre part la viabilité des DC qui au contraire diminue avec le temps d'incubation (Figure 2).

**Exemple 5 : Mise en évidence de l'hétérogénéité des réponses des lignées de lymphocytes T CD4+ induits contre une protéine.**

**1) Matériel et méthodes**

A partir des PBMC d'un seul donneur, des lignées de lymphocytes T CD4+ dirigés contre l'OVA ont été obtenues dans 20 puits différents comme décrit à l'exemple 3 et leur spécificité a été évaluée en ELISPOT comme décrit à l'exemple 3.

**2) Résultats**

Les résultats (Figure 6) montrent une forte hétérogénéité des valeurs observées aussi bien dans les puits où les DC ont été chargées avec la protéine que lorsqu'elles ne sont pas chargées. En revanche, les triplicats sont très homogènes démontrant que les fluctuations observées résultent des différences entre les lignées de lymphocytes T CD4+ et non du manque de précision du test de spécificité. Chaque lignée de lymphocytes T CD4+ se comporte de manière indépendante des autres lignées. Alors que tous les puits ont été ensemencés dans les mêmes conditions, on observe trois catégories de lignées de lymphocytes T CD4+:

1. les lignées de lymphocytes T CD4+ spécifiques de la protéine : ces lignées présentent un nombre de spots en présence de DC chargées avec l'OVA qui est plus du double de celui obtenu en présence de DC non chargées, avec un nombre de spots minimum de 15 pour 10 000 lymphocytes T CD4+. C'est le cas des lignées 1 à 9.

2. les lignées de lymphocytes T CD4+ qui présentent un nombre de spot inférieur au seuil minimum de spots et qui sont non spécifiques de l'OVA. Il s'agit des lignées 10 à 17.

3. les lignées de lymphocytes T CD4+ qui dépassent le nombre de spot minimum mais qui présentent un nombre de spots en présence de DC chargées avec l'OVA qui est moins du double de celui obtenu en présence de DC non chargées. Ces lignées de lymphocytes T CD4+ (18 à 20) sont également non spécifiques de l'OVA alors qu'elles donnent un signal important en présence de DC chargées avec l'OVA.

Aussi, alors que tous les puits ont été ensemencés dans les mêmes conditions, on observe que toutes les lignées ne sont pas spécifiques de l'OVA.

**Exemple 6: Comparaison de l'immunogénicité de protéines immunogènes et de protéines non-immunogènes.**

**1) Matériel et méthodes**

Des lignées de lymphocytes T CD4+ dirigés contre différentes protéines immunogènes (OVA et KLH) et non-immunogènes chez l'homme (insuline humaine, anti-trypsine humaine, anti-thrombine humaine) ont été obtenues à partir de PBMC de trois donneurs sains et la spécificité de chaque lignée a été évaluée en ELISPOT comme décrit à l'exemple 3.

**2) Résultats**

La méthode d'évaluation de l'immunogénicité a été testée sur des protéines immunogènes (OVA et KLH) et sur des protéines connues comme n'étant pas immunogènes chez l'homme (insuline humaine, anti-trypsine humaine, anti-thrombine humaine). Des lignées de lymphocytes T CD4+ dirigées contre ces protéines ont été obtenues chez trois donneurs différents (Tableau I).

**Tableau I: Evaluation de l'immunogénicité de protéines immunogènes et non-immunogènes**

| Protéine | Intensité de la réponse | | | | | |
|---|---|---|---|---|---|---|
| | % puits positifs | Moyenne des différences (*Ai-Bi*) | Quotient des moyennes | Moyenne des valeurs *Ai* | Moyenne des valeurs *Bi* | Moyenne des quotients *Ai/Bi* |
| **OVA** | 23 | 6,51 | 4,94 | 14 | 7 | 1,78 |
| **KLH** | 50 | 24,40 | 21,26 | 29 | 4 | 8,15 |
| **insuline** | 0 | -1,23 | 1,14 | 6 | 7 | 0,84 |
| **anti-trypsine** | 0 | 1,18 | 1,57 | 13 | 11 | 1,10 |
| **anti-thrombine** | 3 | 3,20 | 1,22 | 9 | 7 | 1,18 |

Pour chacune des protéines, le nombre moyen de spots par puits, en présence de DC chargées en protéine (Valeur *Ai* ) et en présence de DC non-chargées (Valeur *Bi*) a été calculé pour chacune des *n* lignées de lymphocytes T (12 < *n* < 20) obtenues pour les trois donneurs (OVA : 55 lignées; KLH : 51 lignées; Insuline: 53 lignées; Anti-trypsine: 51 lignées; Anti-thrombine : 40 lignées). Chaque lignée a été testée en triplicat. Les résultats correspondent aux moyennes de l'ensemble des puits testés (3 puits par lignée ; OVA : 55 lignées ; KLH : 51 lignées ; Insuline : 53 lignées ; Anti-trypsine : 51 lignées ; Anti-thrombine : 40 lignées).

Le pourcentage de puits positifs (2ième colonne du Tableau I) correspond au nombre de puits comprenant un nombre moyen de spots en présence de DC chargées en protéine (valeur *Ai*) qui est supérieur à 15 spots/ 10 000 lymphocytes et au moins le double du nombre moyen de spots en présence de DC non-chargées (Valeur *Bi*) sur l'ensemble des puits testés.

La moyenne des valeurs *Ai* (5ième colonne du Tableau I) est calculée à partir des nombres de spots/puits en présence de DC chargées en protéine (valeurs *Ai*) mesurées sur l'ensemble des puits testés.

La moyenne des valeurs *Bi* (6ième colonne du Tableau I) est calculée à partir des nombres de spots/puits en présence de DC non-chargées en protéine (valeurs *Bi*) mesurées sur l'ensemble des puits testés.

Le quotient des moyennes (moyenne des *Ai*/moyenne des *Bi* ; 4ième colonne du Tableau I)) est calculé directement à partir des valeurs précédentes.

La moyenne des différences (3iéme colonne du Tableau I) est calculée à partir des valeurs (*Ai-Bi*) mesurées sur l'ensemble des puits testés.

La moyenne des quotients (7ième colonne du Tableau I) est calculée à partir des quotients (*Ai/Bi*) mesurées sur l'ensemble des puits testés.

Les résultats (Tableau I) obtenus montrent que pour la KLH, et l'OVA qui sont immunogènes les intensités de réponse spécifique sont élevées alors qu'elles sont faibles pour les autres protéines qui ne sont pas immunogènes.

**Exemple 7:** Comparaison de l'immunogénicité de protéines thérapeutiques

**1) Matériel et méthodes**

Des lignées de lymphocytes T CD4+ dirigées contre différentes protéines thérapeutiques (etanercept, infliximab et rituximab) et deux protéines immunogènes de référence (KLH, anticorps monoclonal murin dénommé M$\alpha$23) ont été obtenues à partir de PBMC de quatre donneurs sains et la spécificité de chaque lignée a été évaluée en ELISPOT comme décrit à l'exemple 3.

**2) Résultats**

L'etanercept, l'infliximab et le rituximab sont des protéines thérapeutiques actuellement utilisées dans des traitements chez l'homme. Leur degré d'immunogénicité mesuré par le pourcentage de sujets présentant des anticorps spécifiques apparait comme variable (Données publiées par l'Agence Européenne des médicaments (EMEA) http://www.emea.europa.eu/ et Tableau II). L'immunogénicité de ces trois protéines a été évaluée par la méthode de l'invention sur quatre donneurs sains, en comparaison avec des protéines immunogènes (KLH et un anticorps monoclonal murin M$\alpha$23).

**Tableau II: Evaluation de l'immunogénicité de protéines thérapeutiques : intensité de la réponse**

| Protéine | Type | Intensité de la réponse | | | | | Immunogénicité* rapportée par l'EMEA |
|---|---|---|---|---|---|---|---|
| | | % puits positifs | moyenne des valeurs $Ai$ | moyenne des valeurs $Bi$ | Moyenne des différences ($Ai$-$Bi$) | Moyenne des quotients $Ai$/$Bi$ | |
| KLH | Protéine non-humaine | 98% | 101 | 7 | 94 | 23,4 | forte |
| Mα23 | Anticorps monoclonal murin | 48% | 31 | 12 | 19 | 5,1 | forte |
| Etanercept | Protéine de fusion humaine | 0% | 9 | 8 | 0 | 0,7 | 1-11% |
| Rituximab | Anticorps chimérique Humain-murin | 7% | 8 | 12 | 0 | 0,8 | 30% |
| Infliximab | Anticorps chimérique Humain-murin | 1% | 14 | 13 | 1 | 1,3 | 13-44% |
| * pourcentage d'individus présentant des anticorps spécifiques. | | | | | | | |

EP 2 376 925 B1

**Tableau III: Evaluation de l'immunogénicité de protéines thérapeutiques : fréquence de répondeurs**

| Protéine | Fréquence de répondeurs (%) |
|---|---|
| KLH | 100 % |
| Mα23 | 100 % |
| Etanercept | 0 % |
| Rituximab | 75 % |
| Infliximab | 25 % |

Les résultats montrent que les protéines témoin (KLH et anticorps murin) qui sont connues comme étant immunogènes induisent des intensités de réponse élevées avec une fréquence de 100 % de répondeurs. Le rituximab qui est connu pour donner naissance à des anticorps chez 30 % des patients traités induit des peu de spots mais présente une fréquence de 75 % de répondeurs. Pour l'infliximab qui est connu pour être immunogène chez 13-44 % des patients, la fréquence de répondeurs et l'intensité de la réponse est encore diminuée. Enfin, l'etanercept qui est peu immunogène n'induit pas de lignées de lymphocytes T CD4+ spécifiques. Les valeurs d'immunogénicité obtenues par le procédé de l'invention sont de deux types. Elles portent sur l'intensité de la réponse observée *in vitro* et sur la fréquence de répondeurs. Les fréquences de répondeurs sont corrélées avec les valeurs obtenues cliniquement.

## Exemple 8 : Evaluation de l'immunogénicité d'un mélange de protéines

La méthode d'évaluation de l'immunogénicité selon l'invention a été testée sur un mélange de protéines de manière à évaluer si le mélange ne perturbe pas l'immunogénicité de chaque protéine. Un mélange équimolaire de la KLH qui est très immunogène et de l'insuline humaine qui ne l'est pas, a été incubé avec des cellules dendritiques autologues pour stimuler des lymphocytes T CD4+, selon le protocole décrit à l'exemple 3. Les lignées de lymphocytes T ainsi obtenues ont été testées en ELISpot IFN-γ vis-à-vis de cellules dendritiques seules, de cellules dendritiques chargées avec la KLH et de cellules dendritiques chargées avec l'insuline humaine, selon le protocole décrit à l'exemple 3. L'expérience a été réalisée sur 4 donneurs différents. L'immunogénicité de chacune des protéines du mélange a été calculée comme décrit à l'exemple 6.

**Tableau IV : Evaluation de l'immunogénicité *in vitro* de protéines thérapeutiques au moyen d'un mélange de protéines**

| Protéine | Puits positifs (%) | Moyenne de Spots | | | | |
|---|---|---|---|---|---|---|
| | | Moyenne des différences (Ai-Bi) | Quotient des moyennes Ai/Bi | Moyenne des valeurs Ai | Moyenne des valeurs Bi | Moyenne des quotients Ai/Bi |
| KLH | 94 | 106 | 4 | 132 | 31 | 13 |
| insuline | 0 | -2 | 1 | 26 | 31 | 1 |
| Valeurs A (+ protéine) Valeurs B (sans protéine) | | | | | | |

Les résultats obtenus montrent que la KLH induit des intensités de réponse élevées (Tableau IV) avec une fréquence de répondeurs de 100 %. L'insuline ne donne aucune réponse spécifique dans aucun des donneurs. Les résultats obtenus sont donc très comparables à ceux obtenus avec les deux protéines séparément. Ceci montre donc que l'immunogénicité de protéines différentes peut être testée alors que ces protéines sont mélangées.

## Exemple 9 : Evaluation de l'immunogénicité d'un anticorps humain

Cette étude supplémentaire a été réalisée sur un anticorps humain (Adalimumab) connu comme pouvant être immunogène chez l'homme. En effet, les réponses en anticorps spécifiques observées chez les sujets traités avec l'Adalimumab peuvent concerner jusqu'à 87 % des patients. (Tableaux V).

Des lignées de lymphocytes T CD4+ dirigées contre l'Adalimumab et contre une protéine de référence (KLH) ont été obtenues à partir de PBMC de neufs donneurs sains et la spécificité de chaque lignée a été évaluée par Elispot IFN-γ comme décrit aux exemples précédents.

**Tableau V : Evaluation de l'immunogénicité des protéines thérapeutiques: intensité de la réponse**

| Protéine | Type | puits positifs (%) | Moyenne de Spots | | | | | Immunogénicité rapportée dans les études cliniques |
|---|---|---|---|---|---|---|---|---|
| | | | Moyenne des différences (Ai-Bi) | Quotient des moyennes Ai/Bi | Moyenne des valeurs Ai | Moyenne des valeurs Bi | Moyenne des quotients Ai/Bi | |
| KLH | Protéine Non-humaine | 88 | 215 | 3 | 311 | 97 | 3,64 | forte |
| Adalimumab | Anticorps humain | 13 | -6 | 1 | 85 | 90 | 1,08 | 4 à 87% |

22

**Tableau VI: Evaluation de l'immunogénicité des protéines thérapeutiques: fréquence de répondeurs**

| Protéine | Type | Fréquence de répondeurs |
|---|---|---|
| KLH | Non-humaine | 100% |
| adalimumab | humaine | 67% |

Ces résultats montrent qu'il y a une forte réponse contre la protéine de référence (KLH). L'intensité de réponse mesurée par le pourcentage de puits positifs ou par le nombre de spot (valeurs A) par rapport au témoin sans protéine (valeurs B), est très élevée. Tous les donneurs sont répondeurs.

L'anticorps humain Adalimumab induit une réponse d'intensité faible mais qui est observée chez 67 % des donneurs. Ce pourcentage de répondeurs est en accord avec les observations faites en clinique.

L'ensemble de ces résultats montre que le procédé de l'invention permet d'évaluer l'immunogénicité des protéines thérapeutiques et qu'il fournit une information sur les risques de réponses chez l'homme. En effet :

- une intensité forte et une fréquence de répondeurs élevées traduisent un risque élevé de réponse immunitaire forte.
- une intensité faible et une fréquence forte traduisent un risque élevé de réponse mais de moindre intensité.
- une intensité forte et une fréquence faible traduisent un risque élevé de réponse pour un nombre limité de sujets. Il s'agit d'une population à risque.
- une intensité faible et une fréquence de répondeurs faible traduisent un risque faible d'immunogénicité.

**Exemple 10: Evaluation de la fréquence de lymphocytes T spécifiques des protéines thérapeutiques**

Le procédé de l'invention permet également d'évaluer la fréquence de lymphocytes T CD4+ spécifiques de la protéine thérapeutique parmi les lymphocytes T CD4+ du donneur.

En effet, les résultats obtenus montrent que parmi tous les puits qui ont été stimulés par la protéine, il existe des puits qui ne donnent pas naissance à des lignées de lymphocytes T CD4+ spécifiques de la protéine. Ceci s'explique par le fait que lors de la distribution des lymphocytes T CD4+ issus du donneur dans les puits de culture, ces puits n'ont pas reçu de lymphocytes T CD4+ spécifiques. La fréquence de ces lymphocytes T CD4+ spécifiques est très faible au point que moins d'une cellule par puits est distribuée. La distribution suit la loi de Poisson, également appelée loi des événements rares.

Très précisément, la répartition des lymphocytes T CD4+ spéci - fiques suit une loi de poisson de paramètre $\lambda$ où $\lambda$ est le nombre moyen de lymphocytes T CD4+ spécifiques par puits.

La probabilité P(X=k) qu'il y ait k lymphocytes T CD4+ spécifiques dans un puits est donnée par la formule : $P(X=k) = e^{-\lambda} \lambda^k/K!$.

On peut alors calculer la probabilité qu'un puits ne contienne pas de cellules spécifiques en prenant la valeur k=0 cette valeur est $P(X=0) = e^{-\lambda}$.

On en déduit la valeur de $\lambda = \ln(P(X=0))$ qui est fonction du nombre de puits négatifs. Le nombre de puits négatifs est en effet une estimation de P(X=0). Ce nombre de puits négatifs se calcule aisément. A partir du nombre de lymphocytes T CD4+ distribuées par puits et de la valeur calculée pour $\lambda$, on remonte au nombre de lymphocytes T CD4+ spécifiques par nombre de lymphocytes T CD4+ distribuées. Ce calcul a été effectué pour plusieurs des protéines précédemment étudiées (Tableau VII). La fréquence moyenne est exprimée en nombre de lymphocytes T CD4+ spécifiques par millions de lymphocytes T CD4+.

**Tableau VII: Exemples de fréquence des lymphocytes T CD4+ spécifiques de protéines thérapeutiques**

| Protéine | Fréquence de lymphocytes T CD4 |
|---|---|
| KLH | 15,98 |
| M$\alpha$23 | 2,26 |
| Rituximab | 0,41 |
| Adalimumab | 0,44 |

**Revendications**

1. Procédé d'évaluation de l'immunogénicité de protéines, comprenant au moins les étapes suivantes:

a) l'activation de lymphocytes T CD4+ d'au moins un donneur par co-culture, dans au moins dix récipients de culture indépendants (n≥ 10) pour chaque donneur, de lymphocytes T CD4$^+$ de chaque donneur avec des cellules dendritiques matures autologues chargées avec une protéine à tester, obtenues à partir de cellules dendritiques immatures qui ont été chargées avec ladite protéine et maturées simultanément avec un agent de maturation,
b) la mesure de l'activation desdits lymphocytes T CD4+ vis-à-vis de cellules dendritiques immatures autologues chargées avec ladite protéine (valeur $Ai$ avec $1 \leq i \leq n$) et la mesure en parallèle de l'activation desdits lymphocytes T CD4+ vis-à-vis de cellules dendritiques immatures autologues non-chargées (valeur $Bi$ avec $1 \leq i \leq n$), pour chacune des $n$ cultures de lymphocytes T CD4$^+$ de chaque donneur de l'étape a), et
c) le calcul de la valeur de l'immunogénicité de ladite protéine par comparaison de l'ensemble des valeurs $Ai$ avec l'ensemble des valeurs $Bi$, obtenues pour l'ensemble des cultures de lymphocytes T CD4$^+$ des donneurs de l'étape a).

2. Procédé selon la revendication 1, **caractérisé en ce que** ledit agent de maturation est le LPS.

3. Procédé selon la revendication 1 ou 2, **caractérisé en ce que** lesdites cellules dendritiques matures sont obtenues à partir de cellules dendritiques immatures qui ont été chargées avec la protéine et maturées simultanément avec l'agent de maturation pendant au moins 4 heures.

4. Procédé selon l'une quelconque des revendications 1 à 3, **caractérisé en ce que** l'étape a) comprend au moins une restimulation des lymphocytes T CD4+ par l'ajout à la co-culture de cellules présentatrices d'antigène autologues chargées avec ladite protéine à tester, lesdites restimulations étant effectuées tous les 5 à 7 jours, la première étant effectuée après au moins 5 jours de co-culture.

5. Procédé selon l'une quelconque des revendications 1 à 4, **caractérisé en ce que** la mesure de l'activation des lymphocytes T CD4$^+$ comprend la mesure de la prolifération desdits lymphocytes T CD4+ ou bien la mesure de la production de cytokine(s) ou de l'expression de marqueur(s) d'activation par lesdits lymphocytes T CD4+.

6. Procédé selon l'une quelconque des revendications 1 à 5, **caractérisé en ce que** le calcul de la valeur de l'immunogénicité de la protéine (étape c)) comprend le calcul de l'intensité de la réponse T CD4+ spécifique.

7. Procédé selon la revendication 6, **caractérisé en ce que** l'intensité de la réponse T CD4+ spécifique est exprimée par l'une des valeurs suivantes:

a) la fréquence de récipients de culture positifs, correspondants à ceux dont la valeur $Ai$ est supérieure au bruit de fond et au moins le double de la valeur $Bi$,
b) la moyenne des différences entre les valeurs $Ai$ et $Bi$, la moyenne des quotients $Ai$ sur $Bi$ ou le quotient de la moyenne des valeurs $Ai$ sur la moyenne des valeurs $Bi$, l'intensité étant significative lorsque la moyenne des différences entre les valeurs $Ai$ et $Bi$ est supérieure au bruit de fond ou bien la moyenne des quotients $Ai$ sur $Bi$ ou le quotient de la moyenne des valeurs $Ai$ sur la moyenne des valeurs $Bi$ est supérieur ou égal à 2, ou
c) la fréquence de lymphocytes T CD4+ spécifiques de ladite protéine parmi les lymphocytes T CD4+ du donneur.

8. Procédé selon l'une quelconque des revendications 1 à 7, **caractérisé en ce que** ladite protéine est une protéine thérapeutique.

9. Procédé selon l'une quelconque des revendications 1 à 8, **caractérisé en ce que** ladite protéine est incluse dans un mélange de protéines différentes.

10. Procédé selon l'une quelconque des revendications 1 à 9, **caractérisé en ce que** ledit donneur est naïf vis-à-vis de ladite protéine.

11. Procédé selon l'une quelconque des revendications 1 à 10, **caractérisé en ce que** les étapes a) à c) sont réalisées en parallèle sur des cultures issues d'une collection de donneurs tels que les fréquences des allèles HLA-DR dans la collection de donneurs sont proches de celles rencontrées dans la population à étudier, et l'étape c) comprend le calcul de l'intensité de la réponse T CD4+ et de la fréquence d'individus répondeurs dans la collection de donneurs.

**12.** Procédé selon l'une quelconque des revendications 1 à 11, **caractérisé en ce que** pour comparer l'immunogénicité de plusieurs protéines, les étapes a) à c) sont réalisées successivement ou simultanément avec les différentes protéines à tester puis les valeurs d'immunogénicité obtenues à l'étape c) sont comparées entre elles.

**13.** Procédé selon la revendication 12, **caractérisé en ce qu'**il comprend la comparaison de l'immunogénicité de variants d'une protéine améliorés par évolution dirigée.

**14.** Procédé selon la revendication 12, **caractérisé en ce qu'**il comprend la comparaison de l'immunogénicité d'au moins une protéine modifiée avec celle d'une protéine de référence non-modifiée.

**15.** Procédé selon la revendication 11 ou la revendication 12, **caractérisé en ce qu'**il comprend la comparaison de l'immunogénicité d'une même protéine à différentes étapes de son processus de fabrication, selon son lot de fabrication, selon sa formulation ou son procédé de production.

**16.** Utilisation du procédé selon l'une quelconque des revendications 1 à 15 pour cribler et sélectionner des protéines thérapeutiques possédant une immunogénicité désirée, pour contrôler l'immunogénicité d'une protéine thérapeutique au cours de sa production ou de sa formulation, ou pour comparer l'immunogénicité d'une protéine thérapeutique dans différentes populations d'individus.

**Patentansprüche**

**1.** Verfahren zur Evaluierung der Immunogenität von Proteinen, umfassend wenigstens die folgenden Schritte:

a) Aktivierung von CD4$^+$-T-Lymphozyten von wenigstens einem Spender durch Cokultivieren, in wenigstens zehn unabhängigen Kulturgefäßen ($n \geq 10$) für jeden Spender, von CD4$^+$-T-Lymphozyten von jedem Spender mit autologen reifen dendritischen Zellen, die mit einem zu testenden Protein beladen sind, erhalten aus unreifen dendritischen Zellen, die mit dem Protein beladen und gleichzeitig mit einem Reifungsmittel gereift wurden,
b) Messung der Aktivierung der CD4$^+$-T-Lymphozyten gegenüber autologen unreifen dendritischen Zellen, die mit diesem Protein beladen sind (Wert $Ai$ mit $1 \leq i \leq n$), und parallel Messung der Aktivierung der CD4$^+$-T-Lymphozyten gegenüber nicht-beladenen autologen unreifen dendritischen Zellen (Wert $Bi$ mit $1 \leq i \leq n$) für jede der $n$ Kulturen von CD4$^+$-T-Lymphozyten von jedem Spender von Schritt a), und
c) Berechnung des Immunogenitätswerts des Proteins durch Vergleich der Gesamtheit der Werte $Ai$ mit der Gesamtheit der Werte $Bi,$ die für die Gesamtheit der Kulturen von CD4$^+$-T-Lymphozyten der Spender von Schritt a) erhalten wurden.

**2.** Verfahren nach Anspruch 1, **dadurch gekennzeichnet, dass** das Reifungsmittel LPS ist.

**3.** Verfahren nach Anspruch 1 oder 2, **dadurch gekennzeichnet, dass** die reifen dendritischen Zellen aus unreifen dendritischen Zellen erhalten sind, die mit dem Protein beladen wurden und gleichzeitig mit dem Reifungsmittel wenigstens 4 Stunden lang gereift wurden.

**4.** Verfahren nach einem der Ansprüche 1 bis 3, **dadurch gekennzeichnet, dass** Schritt a) wenigstens eine Restimulation der CD4$^+$-T-Lymphozyten durch Zusetzen von autologen antigenpräsentierenden Zellen zu der Cokultur, die mit dem zu testenden Protein beladen sind, wobei die Restimulationen alle 5 bis 7 Tage erfolgen, wobei die erste nach wenigstens 5 Tagen Cokultur erfolgt.

**5.** Verfahren nach einem der Ansprüche 1 bis 4, **dadurch gekennzeichnet, dass** die Messung der Aktivierung der CD4$^+$-T-Lymphozyten die Messung der Proliferation der CD4$^+$-T-Lymphozyten oder aber die Messung der Produktion von Cytokin(en) oder der Expression von Aktivierungsmarker(n) durch die CD4$^+$-T-Lymphozyten umfasst.

**6.** Verfahren nach einem der Ansprüche 1 bis 5, **dadurch gekennzeichnet, dass** die Berechnung des Immunogenitätswerts des Proteins (Schritt c)) die Berechnung der Intensität der spezifischen CD4$^+$-T-Antwort umfasst.

**7.** Verfahren nach Anspruch 6, **dadurch gekennzeichnet, dass** die Intensität der spezifischen CD4$^+$-T-Antwort durch einen der folgenden Werte ausgedrückt wird:

a) die Häufigkeit positiver Kulturgefäße, entsprechend denjenigen, bei denen der Wert *Ai* über dem Grundrauschen liegt und mindestens das Doppelte des Werts *Bi* ist,

b) den Mittelwert der Unterschiede zwischen den Werten *Ai* und *Bi,* den Mittelwert der Quotienten *Ai* durch *Bi* oder den Quotienten des Mittelwerts der Werte *Ai* durch den Mittelwert der Werte *Bi,* wobei die Intensität signifikant ist, wenn der Mittelwert der Unterschiede zwischen den Werten *Ai* und *Bi* über dem Grundrauschen liegt oder aber der Mittelwert der Quotienten *Ai* durch *Bi* oder der Quotient des Mittelwerts der Werte *Ai* durch den Mittelwert der Werte *Bi* größer oder gleich 2 ist, oder

c) die Häufigkeit von CD4$^+$-T-Lymphozyten unter den CD4$^+$-T-Lymphozyten des Spenders, die für das Protein spezifisch sind.

8. Verfahren nach einem der Ansprüche 1 bis 7, **dadurch gekennzeichnet, dass** das Protein ein therapeutisches Protein ist.

9. Verfahren nach einem der Ansprüche 1 bis 8, **dadurch gekennzeichnet, dass** das Protein in einer Mischung verschiedener Proteine enthalten ist.

10. Verfahren nach einem der Ansprüche 1 bis 9, **dadurch gekennzeichnet, dass** der Spender hinsichtlich des Proteins naiv ist.

11. Verfahren nach einem der Ansprüche 1 bis 10, **dadurch gekennzeichnet, dass** die Schritte a) bis c) parallel an Kulturen durchgeführt werden, die von einer Reihe von Spendern stammen, derart, dass die Häufigkeiten der HLA-DR-Allelen in der Reihe von Spendern nahe denen liegen, die man in der zu untersuchenden Population findet, und Schritt c) die Berechnung der Intensität der CD4$^+$-T-Antwort und der Häufigkeit von Responderindividuen in der Reihe von Spendern umfasst.

12. Verfahren nach einem der Ansprüche 1 bis 11, **dadurch gekennzeichnet, dass** die Schritte a) bis c) zum Vergleich der Immunogenität mehrerer Proteine nacheinander oder gleichzeitig mit den verschiedenen zu testenden Proteinen durchgeführt werden, und die in Schritt c) erhaltenen Immunogenitätswerte dann miteinander verglichen werden.

13. Verfahren nach Anspruch 12, **dadurch gekennzeichnet, dass** es den Vergleich der Immunogenität von durch gerichtete Evolution verbesserten Varianten eines Proteins umfasst.

14. Verfahren nach Anspruch 12, **dadurch gekennzeichnet, dass** es den Vergleich der Immunogenität wenigstens eines modifizierten Proteins mit der eines nicht modifizierten Referenzproteins umfasst.

15. Verfahren nach Anspruch 11 oder Anspruch 12, **dadurch gekennzeichnet, dass** es den Vergleich der Immunogenität desselben Proteins bei unterschiedlichen Stufen seines Herstellungsprozesses, nach seiner Herstellungscharge, nach seiner Formulierung oder seinem Produktionsverfahren umfasst.

16. Verwendung des Verfahrens nach einem der Ansprüche 1 bis 15 zum Screenen und Selektionieren von therapeutischen Proteinen, die eine gewünschte Immunogenität besitzen, zum Prüfen der Immunogenität eines therapeutischen Proteins während seiner Produktion oder seiner Formulierung, oder zum Vergleichen der Immunogenität eines therapeutischen Proteins in verschiedenen Populationen von Individuen.

**Claims**

1. A method for evaluating the immunogenicity of proteins, comprising at least the following steps:

a) activating CD4+ T lymphocytes of at least one donor by coculturing CD4+ T lymphocytes of each donor with autologous mature dendritic cells loaded with a test protein, obtained from immature dendritic cells which have been loaded with the protein and matured simultaneously with a maturation agent, in at least ten independent culture vessels ($n \geq 10$) for each donor,

b) measuring the activation of said CD4+ T lymphocytes with respect to autologous immature dendritic cells loaded with said protein (value *Ai* with $1 \leq i \leq n$) and measuring, in parallel, the activation of said CD4+ T lymphocytes with respect to non-loaded autologous immature dendritic cells (value *Bi* with $1 \leq i \leq n$), for each of the *n* cultures of CD4+ T lymphocytes of each donor of step a), and

c) calculating the value of the immunogenicity of said protein by comparison of all the values *Ai* with all the values *Bi,* obtained for all the cultures of CD4+ T lymphocytes of the donors of step a).

2. The method as claimed in claim 1, **characterized in that** said maturation agent is LPS.

3. The method as claimed in claim 1 or 2, **characterized in that** said mature dendritic cells are obtained from immature dendritic cells which have been loaded with the protein and incubated simultaneously with the maturation agent for at least 4 hours.

4. The method as claimed in any one of claims 1 to 3, **characterized in that** step a) comprises at least one restimulation of the CD4+ T lymphocytes by addition, to the coculture, of autologous antigen-presenting cells loaded with said test protein, said restimulations being carried out every 5 to 7 days, the first being carried out after at least 5 days of coculture.

5. The method as claimed in any one of claims 1 to 4, **characterized in that** the measurement of the activation of the CD4+ T lymphocytes comprises measuring the proliferation of said CD4+ T lymphocytes or else measuring the production of cytokine(s) or the expression of activation marker(s) by said CD4+ T lymphocytes.

6. The method as claimed in any one of claims 1 to 5, **characterized in that** the calculation of the value of the immunogenicity of the protein (step c)) comprises calculating the strength of the specific CD4+ T response.

7. The method as claimed in claim 6, **characterized in that** the strength of the specific CD4+ T response is expressed by one of the following values:

   a) the frequency of positive culture vessels, corresponding to those of which the value *Ai* is greater than the background noise and at least double the value *Bi,*
   b) the mean of the differences between the values *Ai* and *Bi,* the mean of the quotients *Ai* over *Bi* or the quotient of the mean of the values *Ai* over the mean of the values *Bi,*
   the strength being significant when the mean of the differences between the values *Ai* and *Bi* is greater than the background noise, or else the mean of the quotients *Ai* over *Bi* or the quotient of the mean of the values *Ai* over the mean of the values *Bi* is greater than or equal to 2, or.
   c) the frequency of CD4+ T lymphocytes specific for said protein among the CD4+ T lymphocytes of the donor.

8. The method as claimed in any one of claims 1 to 7, **characterized in that** said protein is a therapeutic protein.

9. The method as claimed in any one of claims 1 to 8, **characterized in that** said protein is included in a mixture of different proteins.

10. The method as claimed in any one of claims 1 to 9, **characterized in that** said donor is naive with respect to said protein.

11. The method as claimed in any one of claims 1 to 10, **characterized in that** steps a) to c) are carried out in parallel on cultures derived from a collection of donors such that the frequencies of the HLA-DR alleles in the collection of donors are close to those encountered in the population to be studied, and step c) comprises calculating the strength of the CD4+ T response and the frequency of responder individuals in the collection of donors.

12. The method as claimed in any one of claims 1 to 11, **characterized in that**, for comparing the immunogenicity of several proteins, steps a) to c) are carried out successively or simultaneously with the various test proteins and then the immunogenicity values obtained in step c) are compared with one another.

13. The method as claimed in claim 12, **characterized in that** it comprises comparing the immunogenicity of variants of a protein which have been improved by directed evolution.

14. The method as claimed in claim 12, **characterized in that** it comprises comparing the immunogenicity of at least one modified protein with that of an unmodified reference protein.

15. The method as claimed in claim 11 or claim 12, **characterized in that** it comprises comparing the immunogenicity of the same protein at various steps of its production process, according to its production batch, according to its formulation or its method of production.

16. The use of the method as claimed in any one of claims 1 to 15, for screening for and selecting therapeutic proteins having a desired immunogenicity, for testing the immunogenicity of a therapeutic protein during its production or its formulation, or for comparing the immunogenicity of a therapeutic protein in various populations of individuals.

**FIGURE 1**

**FIGURE 2**

**A**

**B**

**FIGURE 3**

**FIGURE 4**

**FIGURE 5**

**FIGURE 6**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1512004 A
- EP 1483581 A
- EP 1073754 A

**Littérature non-brevet citée dans la description**

- **A.S. DE GROOT ; D. W. SCOTT.** *TRENDS in Immunology,* 27 Octobre 2007, vol. 28
- **U. KRAGH-HANSEN.** *Pharmacol. Rev.,* 1981, vol. 33, 17-53
- **H. BUNDGAARD ; J. HANSEN.** *J. Pharm. Pharmacol.,* 1982, vol. 34, 304-309
- **LAFAYE ; C. LAPRESLE.** *J. Clin. Invest.,* 1988, vol. 82, 7-12
- **BRANDER et al.** *The Journal of Immunology,* 1995, vol. 155, 2670-2678
- **P; J. P. THYSSE ; H.I. MAIBACH.** *Contact Dermatitis,* 2008, vol. 59, 195-202
- **STEINMAN.** *J. Exp. Med.,* 1973, vol. 137, 1142-1162
- **JACQUEMIN et al.** *Blood,* 1998, vol. 92, 496-506
- **BRAY et al.** *Am. J. Hematol.,* 1993, vol. 42, 375-379
- **HERVE et al.** *Mol. Immunol.,* 1997, vol. 34, 157-163
- **YEUNG et al.** *J. Immunol.,* 2004, vol. 172, 6658-6665
- **ADORINI et al.** *J. Exp. Med.,* 1988, vol. 168, 2091-2104
- **STURNIOLO et al.** *Nat. Biotechnol.,* 1999, vol. 17, 555-561
- **KOREN et al.** *lin. Immunol.,* 2007, vol. 124, 26-32
- **DESMET et al.** *Proteins,* 2005, vol. 58, 53-69
- **WANG et al.** *PLoS Comput. Biol.,* 2008, vol. 4, 1000048
- **CASTELLI et al.** *Eur. J.Immunol.,* 2007, vol. 37, 1513-1523
- **TANGRI et al.** *J. Immunol.,* 2005, vol. 174, 3187-3196
- **STICKLER et al.** *Environ. Health Perspect.,* 2003, vol. 111, 251-254

- **JABER, A. ; M. BAKER.** *J Pharm. Biomed Anal.,* 2007, vol. 43, 1256-1261
- **GAMMON et al.** *Immunol. Rev.,* 1987, vol. 98, 53-73
- **ADORINI et al.** *J. Exp.Med.,* 1988, vol. 168, 2091-2104
- **JABER, A. ; M. BAKER.** *J. Pharm. Biomed Anal.,* 2007
- **SCHLIENGER et al.** *Blood,* 2000, vol. 96, 3490-3498
- **TRÉMEAU et al.** *FEBS Lett.,* 1986, vol. 208 (2), 236-240
- **CASTELLI E.** *European Journal of Immunology,* 2007, vol. 37, 1513-1523
- **CASTELLI et al.** *European Journal of Immunology,* 2007, vol. 37, 1513-1523
- **CASTELLI et al.** *European Journal of Immunology,* 2008, vol. 38, 1-11
- **FREDERICK M. AUSUBEL.** Current Protocols in Molecular Biology. Wiley and Sons Inc
- **JOHN E. COLIGAN et al.** Current Protocols in Immunology. Wiley and Sons Inc
- **SAMBROOK et al.** Molecular Cloning: A Laboratory Manual. Cold Spring Harbor Laboratory Press
- **R. I. FRESHNEY.** Culture Of Animal Cells. Alan R. Liss, Inc,
- Immobilized Cells And Enzymes. IRL Press
- A Practical Guide To Molecular Cloning. **B. PERBAL et al.** Methods In ENZYMOLOGY. Academic Press, Inc, 1984, vol. 154, 155
- Gene Expression Technology. METHODS IN ENZYMOLOGY. vol. 185
- Gene Transfer Vectors For Mammalian Cells. Cold Spring Harbor Laboratory, 1987
- Immunochemical Methods In Cell And Molecular Biology. Academic Press, 1987
- Handbook Of Experimental Immunology. 1986, vol. I-IV